(19) Europäisches Patentamt
European Patent Office
Office européen des brevets

(11) Publication number: 0 265 640
A2

(12) # EUROPEAN PATENT APPLICATION

(21) Application number: 87113159.5

(22) Date of filing: 09.09.87

(51) Int. Cl.[4]: C07D 471/04 , A61K 31/435 , //(C07D471/04,221:00,209:00)

(30) Priority: 10.09.86 US 905897
23.12.86 US 945750

(43) Date of publication of application:
04.05.88 Bulletin 88/18

(84) Designated Contracting States:
AT BE CH DE ES FR GB GR IT LI LU NL SE

(71) Applicant: SANDOZ AG
Lichtstrasse 35
CH-4002 Basel(CH)

(84) BE CH ES FR GB GR IT LI LU NL SE

(71) Applicant: SANDOZ-PATENT-GMBH
Humboldtstrasse 3
D-7850 Lörrach(DE)

(84) DE

(71) Applicant: SANDOZ-ERFINDUNGEN
Verwaltungsgesellschaft m.b.H.
Brunner Strasse 59
A-1235 Wien(AT)

(84) AT

(72) Inventor: Anderson, Paul LeRoy
264 Center Grove Road
Randolph, N.J. 07869(US)
Inventor: Kathawala, Faizulla Gulamhusein
39 Woodland Avenue
Mountain Lakes, 07046(US)
Inventor: Paolella, Nicholas Andrew
46 Mt. Pleasant Parkway
Livingston, N.J. 07039(US)
Inventor: Wattanasin, Sompong
30 Beech Street
Stanhipe, N.J. 07874(US)

(54) Azaindole and indolizine derivatives, processes for their production and their use as pharmaceuticals.

(57) Compounds of Formula I:

EP 0 265 640 A2

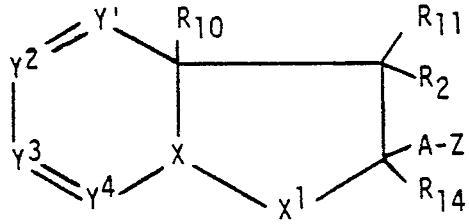

I

wherein:

either one Y is -N-and the remainder are -CH-

X is 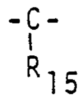 , $X^1$ is 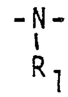

with appropriate bond formation,

$R_1$ is a primary or secondary alkyl, free of asymmetric carbon atoms,

$R_2$ is a substituted or unsubstituted phenyl, primary or secondary $C_{1-6}$ alkyl, free of asymmetric carbon atoms, $C_{3-6}$ cycloalkyl or phenylakyl as defined in the specification,

A is

a)

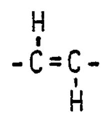

or 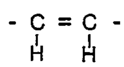

b) $-(CH_2)_n-$

wherein n is l,2 or 3 Z is

a) 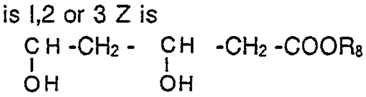

b)

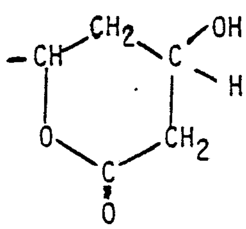

in which $R_8$ is hydrogen, $R_9$ or M

wherein $R_9$ is a physiologically acceptable and hydrolysable ester group, and M is a pharmaceutically acceptable cation; or: each Y is -CH-, X is -N-, $X^1$ is

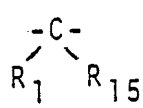

with appropriate bond formation,

$R_1$ and $R_2$ are independently as defined under $R_2$ above and $R_2$ may additionally be hydrogen

A and Z are as defined above

are obtained by multi-step processes and are useful as hypolipoproteinemic and anti-atherosclerotic agents.

## AZAINDOLE AND INDOLIZINE DERIVATIVES, PROCESSES FOR THEIR PRODUCTION AND THEIR USE AS PHARMACEUTICALS.

The invention concerns azaindole and indolizine derivatives, processes for their production, pharmaceutical compositions containing them and their use as pharmaceuticals in particular as hypolipoproteinemic and anti-atherosclerotic agents.

The invention refers more particularly to compounds which may conveniently be represented by Formula I:

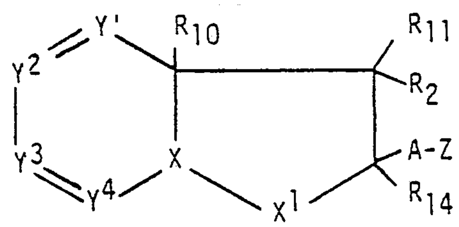

I

wherein:

<u>either</u> one of $Y^1$-$Y^4$ is -N-, and the others are -CH-

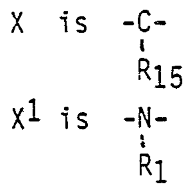

and $R_{10}$ and $R_{15}$, and $R_{11}$ and $R_{14}$ form bonds

$R_1$ is a primary or secondary $C_{1-6}$alkyl, not containing an asymmetric carbon atom;

$R_2$ is

a)

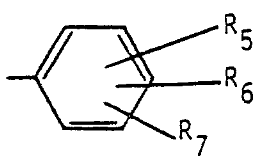

b) a primary or secondary $C_{1-6}$alkyl, not containing an asymmetric carbon atom

c) $C_{3-6}$ cycloalkyl or

d) phenyl-$(CH_2)_m$-

wherein $R_5$ is hydrogen, $C_{1-3}$alkyl, n-butyl, i-butyl, t-butyl, $C_{1-3}$alkoxy, n-butoxy, i-butoxy, trifluoromethyl fluoro, chloro, phenoxy or benzyloxy;

$R_6$ is hydrogen, $C_{1-3}$alkyl, $C_{1-3}$alkoxy, fluoro, chloro, trifluoromethyl, phenoxy or benzyloxy;

$R_7$ is hydrogen, $C_{1-2}$ alkyl, $C_{1-2}$alkoxy, fluoro or chloro;

m is 1, 2 or 3;

with the proviso that not more than one of $R_5$ and $R_6$ is trifluoromethyl, not more than one of $R_5$ and $R_6$ is phenoxy, and not more than one of $R_5$ and $R_6$ is benzyloxy;

<u>OR</u> $Y^1$-$Y^4$ are -CH-
X is -N-

$X^1$ is 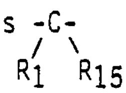

and $R_{14}$ and $R_{15}$, and $R_{10}$ and $R_{11}$ form bonds; each of $R_1$ and $R_2$ is, independently, as defined under $R_2$ above, and $R_2$ may additionally be hydrogen:

A is a) 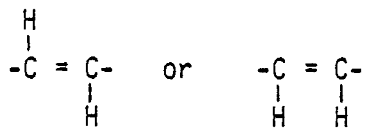 b) $-(CH_2)_n-$

wherein n is 1,2 or 3

Z is a) 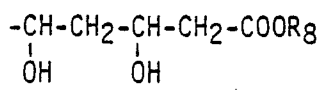 or b) 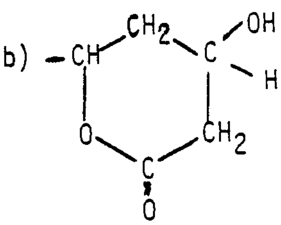

and in which $R_8$ is hydrogen, $R_9$ or M,

wherein $R_9$ is a physiologically acceptable and hydrolysable ester group, and M is a pharmaceutically acceptable cation.

Compounds I may be viewed as consisting of various sub-classes depending upon the definition of their variable portions. Compounds I may be of the following sub-classes depending upon the substituents for Y, X and $X^1$

| Designation | $Y^1$ to $Y^4$ | X= | $X^1$= |
|---|---|---|---|
| Ia | CH | -N- | 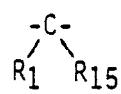 |
| Ib | $Y^1$=N<br>rest = CH | 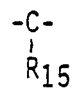 | 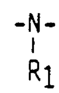 |
| Ic | $Y^2$=N<br>rest = CH | " | " |
| Id | $Y^3$=N<br>rest = CH | " | " |
| Ie | $Y^4$=N<br>rest = CH | " | " |

Compounds I may be further classified according to the nature of Z.

| Designation | Z = | Nature |
|---|---|---|
| I1a → I1e | type a $R_8$ = H | free acid |
| I2a → I2e | type a $R_8$ = $R_9$ | ester |
| I3a → I3e | type a $R_8$ = M | salt |
| I4a → I4e | type b | lactone |

Compounds I may further be viewed as two subclasses depending on the nature of A, i.e. I° where A = a) and $I^x$ where A = b), the former being preferred.

Preferred types of Compounds Ib to Ie may be designated Iα, in which $R_1$ is alkyl and especially methyl or isopropyl; A is a) and $R_2$ is an aryl group ie a) especially p-fluorophenyl, phenyl or 3,5-dimethylphenyl and particularly p-fluorophenyl. Preferred types of Compounds Ia may be designated Iβ in which A is a) and one of $R_1$ and $R_2$ is alkyl, especially methyl or isopropyl and the other is an aryl group ie a) especially p-fluorophenyl, phenyl or 3,5-dimethylphenyl and particularly p-fluorophenyl.

A particular sub-class of Compounds I is Compounds I2′ in which $R_8$ is $R_9''$ ie a $C_{1-4}$ primary alkyl, especially ethyl, i.e. compounds I2a′, I2b′, I2c′ etc.

An example of a compound of Formula I could therefore be a compound I3b° in which A is type a), Z is type a), $Y^1$ = N,

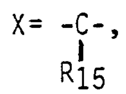

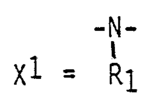

and $R_8$ = M. Likewise the other compounds may be so designated e.g. I4a°, I2d°′ etc.

When $R_8$ is $R_9$, it is preferably ethyl, and when it is M, it is preferably sodium, potassium, magnesium, ammonium or calcium. Monovalent cations especially sodium are preferred.

By the term "physiologically acceptable and hydrolyzable ester group" is meant a group which, together with the -CCO-radical to which it is attached, forms an ester group which is physiologically acceptable and hydrolyzable under physiological conditions to yield a compound of Formula I wherein $R_8$ is hydrogen and an alcohol which itself is physiologically acceptable, i.e., non-toxic at the desired dosage level, and which, preferably, is free of centers of asymmetry. Examples of such groups are $C_{1-3}$alkyl, n-butyl, i-butyl, t-butyl and benzyl, collectively referred to as $R_9'$.

As is self-evident to those in the art, each compound of formula I (and every sub-scope and species thereof) has at least two centers of asymmetry (e.g. the two carbon atoms bearing the hydroxy groups in the structure when Z = a) and the carbon atom bearing the hydroxy group and the carbon atom having the free valence in the structure when Z = b), and these lead (e.g. with two centers) to four stereoisomeric forms (enantiomers) of each compound (two racemates or pairs of diastereoisomers). The preferred compounds have only two such centers of asymmetry and these four stereoisomers may be designated as the R,R; R,S; S,R; and S,S enantiomers, all four stereoisomers being within the scope of this invention.

The preferred stereoisomers of the compounds of formula I having only two centers of asymmetry wherein A is a) and Z is a) are the 3R,5S and 3R,5R isomers and the racemate of which each is a constituent, i.e., the 3R,5S-3S,5R (erythro) and 3R,5R-3S,5S (threo) racemates, with the 3R,5S isomer and the racemate of which it is a constituent being more preferred and the 3R,5S isomer being most preferred.

The preferred stereoisomers of the compounds of formula I having only two centers of asymmetry wherein A is b) and Z is a) are the 3R,5R and 3R,5S isomers and the racemate of which each is a constituent, i.e., the 3R,5R-3S,5S (erythro) and 3R,5S-3S,5R (threo) racemates, with the 3R,5R isomer and the racemate of which it is a constituent being more preferred and the 3R,5R isomer being most preferred.

The preferences set forth in the preceding two paragraphs also apply to the compounds of formula I having more than two centers of asymmetry and represent the preferred configurations of the indicated positions.

The preferred stereoisomers of the compounds of formula I wherein A is a) and Z is b) are the 4R,6S and 4R,6R isomers and the racemate of which each is a constituent, i.e., the 4R,6S-4S,6R (trans lactone) and 4R,6R-4S,6S (cis lactone) racemates, with the 4R,6S isomer and the racemate of which it is a constituent being more preferred and the 4R,6S isomer being most preferred.

The preferred stereoisomers of the compounds of formula I wherein A is b) and Z is b) are the 4R,6R and 4R,6S isomers and the racemate of which each is a constituent, i.e., the 4R, 6R-4S,6S (trans lactone) and 4R, 6S-4S,6R (cis lactone) racemates, with the 4R,6R isomer and the racemate of which it is a constituent being more preferred and the 4R,6R isomer being most preferred.

Compounds of formula I in which A is a), Z is a) and other substituents are as described above, may be prepared by the following reactions, wherein substituents are as defined above,

a) Reducing a compound of formula AII:

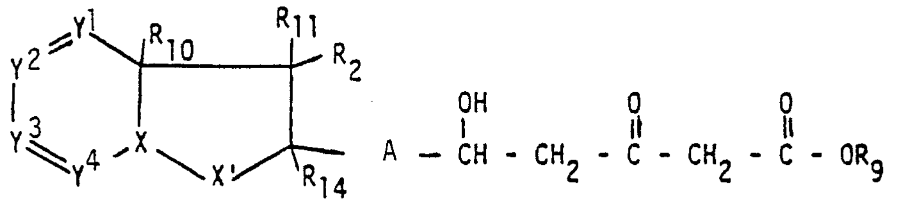

AII

b) Deprotecting a compound of formula BII:

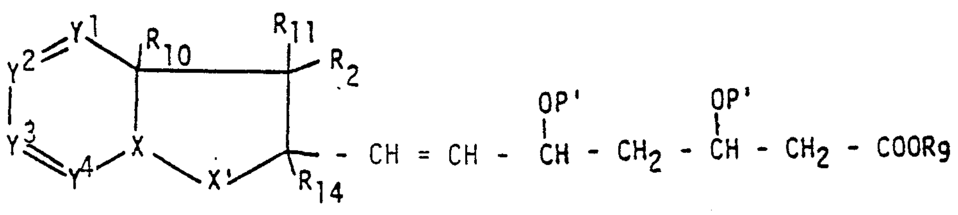

BII

in which P' is a protective group

c) hydrolysing a compound of Formula I in the form of an ester or a lactone or

d) esterifying or lactonising a compound of Formula I in free acid form and when a free carboxyl group is present, recovering the compound obtained in free acid form or in the form of salt.

Processes a and b are particularly suitable when $R_9$ is $R_9'$ and more especially when $R_9$ is $R_9''$, ie a $C_{1-4}$ primary alkyl, especially ethyl.

It will readily be appreciated that the various forms of the compounds of Formula I may be interconverted as indicated in c) and d) above.

In the same way compounds obtained according to a) and b) may be as appropriate, hydrolysed to free acid forms and free acid forms may be esterified or lactonised to produce a desired end-product. The invention thus also provides a process for preparing a compound of Formula I which comprises hydrolysing a compound of Formula I in ester or lactone form or esterifying or lactonising a compound of formula I in free acid form and when a free carboxyl group is present recovering the compound obtained in free acid form or in the form of a salt.

Unless otherwise stated reactions are performed in a manner conventional for the type of reaction involved. Molar ratios and reaction times are as a rule conventional and non-critical and are chosen according to principles well established in the art on the basis of reactions and conditions exmployed.

See for example, U.S. Patent 4,613,610 and WO 86/07054. Unless indicated otherwise, evaporations are done under reduced pressure, drying of extracts is done over anhydrous sodium sulfate, all ratios of liquid mixtures are volume to volume, and moisture-free solvents and dry nitrogen atmosphere are employed for all reactions which are indicated to be carried out under essentially anhydrous conditions. All temperatures are centigrade and room temperature (RT) is 20 to 30 °C, unless indicated otherwise.

In processes in which a medium is employed, it is understood that the medium is an inert solvent under the reaction conditions and is essentially anhydrous, ie moisture-free, if essentially anhydrous conditions are called for.

Reduction according in a) is preferably carried out using a mild reducing agent such as sodium borahydride or, preferably, a complex of t-butylamine and borane in an inert organic solvent such as a lower alkanol, preferably ethanol, conveniently at a temperature of -10 to 30 °C, under an inert atmosphere. When a racemic AII is used, the product is a mixture of all four possible stereoisomers (the threo and erythro racemates).

A stereoselective reduction may otherwise be performed, preferably carried out in three steps. For example in the first step, the compound of formula AII is treated with a trialkyl borane reagent in an inert organic solvent such as tetrahydrofuran/lower alcohol eg methanol mixture (3-4:1) or pure tetrahydrofuran, conveniently at a temperature of 0° to 40 °C under anhydrous conditions. In the second step, for example, the complex is reduced with sodium borahydride, preferably in the same solvent as utilised for the first step, at -100° to -20 °C preferably -90° to -70 °C. In the third step, the product of the second step is, for example, treated with, preferably, anhydrous methanol at 20° to 40 °C. The amount of methanol is not critical. However, a large excess, eg 50-500 moles per mole of compound of formula AII, is typically utilised.

In an alternative method of performing this reduction, the trialkyl borane reagent may be substituted by an equivalent amount of a monoalkoxy dialkyl borane of the formual GK:

GK: $R_{21}$ 0 - B - $(R_{18})_2$

in which $R_{18}$ is a primary or secondary $C_2$-$C_4$alkyl eg ethyl and $R_{21}$ is allyl or a $C_{1-4}$alkyl, preferably not tertiary, eg methyl. Preparation of compounds GK is described by Koster et al, Ann. 1975, 352.

Hydrolysis according to c) is carried out in a manner conventional for such reactions eg employing an inorganic hydroxide such as NaOH or KOH with, if desired, subsequent acidification to give the free acid form. Suitable solvents are mixtures of water and water miscible solvents such as lower alkanols eg methanol or ethanol and reaction conveniently takes place at temperatures from 0° to reflux preferably 0° to 75 °C eg 20° to 70 °C. If it is desired to recover the compound in a salt form corresponding to the cation of the hydroxide employed, then slightly less than equivalent amounts of the latter may be employed. Lactonisation according to d) is carried out in conventional manner eg by heating the corresponding acid in an anhydrous inert organic solvent eg a hydrocarbon such as benzene, toluene or a xylene or mixtures thereof, preferably at temperatures of 75 °C to reflux although more preferably not above 150 °C.

Preferably however, a lactonisation agent, eg a carbodiimide, preferably a water-soluble carbodiimide such as N-cyclohexyl-N'-[2'-(methylmorpholinium)ethyl]carbodiimide p-toluene-sulfonate, in an anhydrous inert organic solvent, eg a halogenated lower alkane, preferably methylene chloride is employed. Reaction temperatures then lie typically between 10° and 35 °C, especially 20° to 25 °C.

As is evident to those in the art, a racemic threo 3,5-dihydroxy-carboxylic acid yields a racemic cis lactone (two stereoisomers) and a racemic erythro 3,5-dihydroxycarboxylic acid yields a racemic trans lactone (two stereoisomers). Likewise if a single enantiomer of the 3,5-dihydroxycarboxylic acid is utilised, a single erantiomer of the lactone is obtained. For example, lactonisation of a 3R, 5S erythro dihydroxy-carboxylic acid yields a 4R, 6S lactone.

Esterification according to d) is conventional, employing eg a large excess of compound $R_9OH$, wherein $R_9$ is as defined above, at eg 20 °C to 40 °C in the presence of a catalytic amount of an acid such as p-toluenesulfonic acid. Alternatively, esterification takes place by first forming the corresponding lactone and reacting this with $M_2^+$ $^-OR_9$ ($M_2^+$ = $Na^+$ or $K^+$) at 0° to 70 °C preferably 20° to 25 °C in an inert organic solvent eg an ether such as tetrahydrofuran or an alcohol of formlula $R_9OH$ if a liquid.

An example of a protecting group in reaction b) is a tri-substitued silyl radical having 2 or 3 bulky radicals; the bulky radicals being independently selected from the group consisting of a) $C_4$-$C_8$ tertiary alkyl, e.g. t-butyl, and b) phenyl which may be unsubstituted or substituted by up to two $C_{1-4}$alkyl, chloro, nitro or trifluoromethyl substituents or monosubstituted in the para-position by phenyl or benzyl, which may be unsubstituted or substituted by one or two of said alkyl, chloro, nitro or trifluoromethyl substituents, and the non-bulky radicals being $C_{1-4}$unbranched alkyl, (a preferred group is diphenyl t-butylsilyl).

Deprotection is carried out in a conventional manner eg by cleavage under mild conditions such as employing eg for removal of a diphenyl-t-butylsilyl a fluoride reagent eg tetra-n-butylammonium fluoride in an anhydrous inert organic medium preferably tetrahydrofuran containing glacial acetic acid at temperatures of 0° to 50 °C especially R.T. The compounds of subgroups Ib to Ie may also be prepared in the form of acid addition salts, such preparation being by conventional means. An example of a suitable acid would be hydrochloric acid.

The required starting materials may be prepared for example as illustrated in the following reaction - schemes, or in the examples hereinafter.

In the following reaction schemes the reagents BIII are obtainable as described in U.S. Patent 4,571,428 (issued Feb. 18, 1986), which patent is incorporated herein by reference. The proportion of threo to erythro forms of Compounds BIII is carried through in products BII and I2(b-e)$^{0}$ $^{1}$. The erythro form is preferred. Also, chiral characteristics of a Compound BIII will result in products of corresponding enantiomeric identity. Hence, particular enantiomers of Compounds I are thus obtainable.

Preparation of Compounds BIII in the 3R, 5S-form are disclosed in US Patent 4,677,211, US Patent 4,613,610 and WO 86/07054, wich are incororated herein by reference.

The Tebbe reagent used in process e2 may be prepared as described in J. Am. Chem. Soc. 102, 3270, (1980) and Pohjala, Heterocycles 2 (1974) 585.

Compounds having the basic indolizine ring structure ie compounds HVII, may be prepared using methods analogous to those described in POHJALA, Acta Chemica Scandinavia B30 (1976) 198 and 512.

An alternative method to that hereafter described for the preparation of the basic azaindole ring structure, e.g. compounds BVII,CV, DIV, FVI and KV, may be found in the Canadian Journal of Chemistry 44 (1966) 2455: Kelly et al, J. Chem. Soc. (1970) 303 and Belgian Patent 840,362.

Abbreviations: NBS N-bromo-succinimide
Et ethyl
THF tetrahydrofuran
LAH lithium aluminium hydride
PTS p-toluene sulfonic acid
LDA lithium di-isopropylamide
N-Bu-Li N-butyl lithium
$SO_2\phi$ benzene sulfonyl

Variables not previously defined: $R_{16}$ is $C_{1-3}$ alkyl, preferably ethyl
$R_{17}$ is lower alkyl eg unbranched $C_{1-4}$alkyl eg ethyl
$R_{19}$ is allyl or $C_{1-4}$ alkyl preferably not tertiary eg methyl
$R_{20}$ is $C_{1-4}$ unbranched alkyl
AK is an alkali source eg LDA or t-butyl-lithium
$AK^+$ is an alkali metal cation eg $Li^+$
V is a leaving group eg higher halo ie chloro, bromo or iodo, preferably chloro and alkyl and aryl sulfonyl radicals eg $C_{1-6}$alkyl or phenyl unsubstituted or mono-substituted by a $C_{1-4}$alkyl such as p-toluene sulfonyl.
T is the leaving portion of an acetylating agent, such as chloro for acetyl chloride, or $O-COCH_3$ for acetic acid anhydride.
$P^2$ is a protective group as P′ previously defined or a tri ($C_{1-4}$)alkylsilyl group eg trimethylsilyl.
Q is an alkali metal salt usually used in situ.
[ ] is an adduct or complex reaction product, which is reacted in a subsequent quenching step to hydrolyse or decompose it.

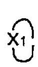 represents compounds in which $Y^1$ is N or $Y^3$ is N.

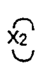 represents compounds in which either $Y^1$, $Y^3$ or $Y^4$ is N.

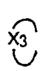 represents compounds in which one of $Y^1$ to $Y^4$ is N.

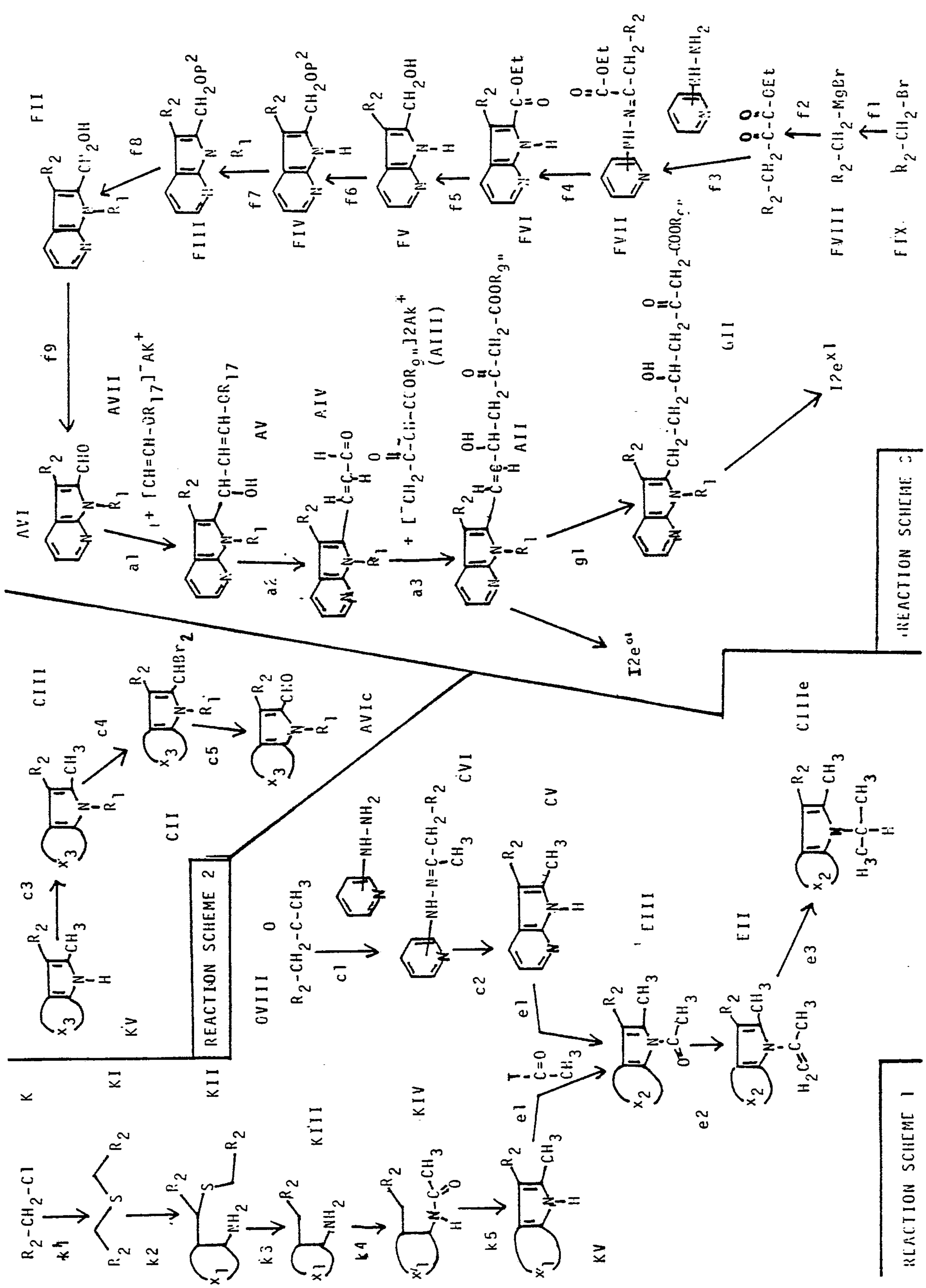
REACTION SCHEME 1
REACTION SCHEME 2
REACTION SCHEME 3

REACTION SCHEME 4

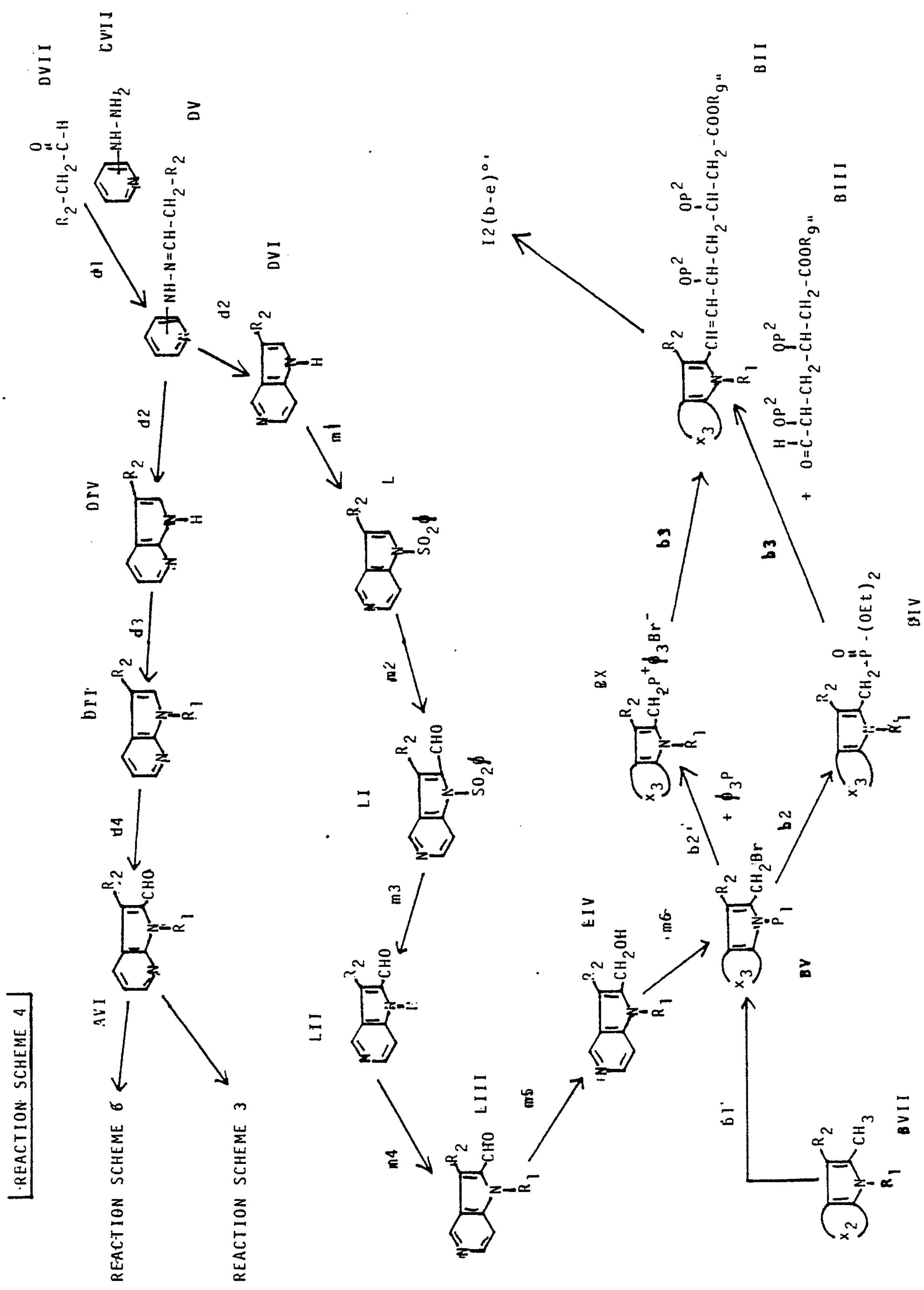

REACTION SCHEME 6

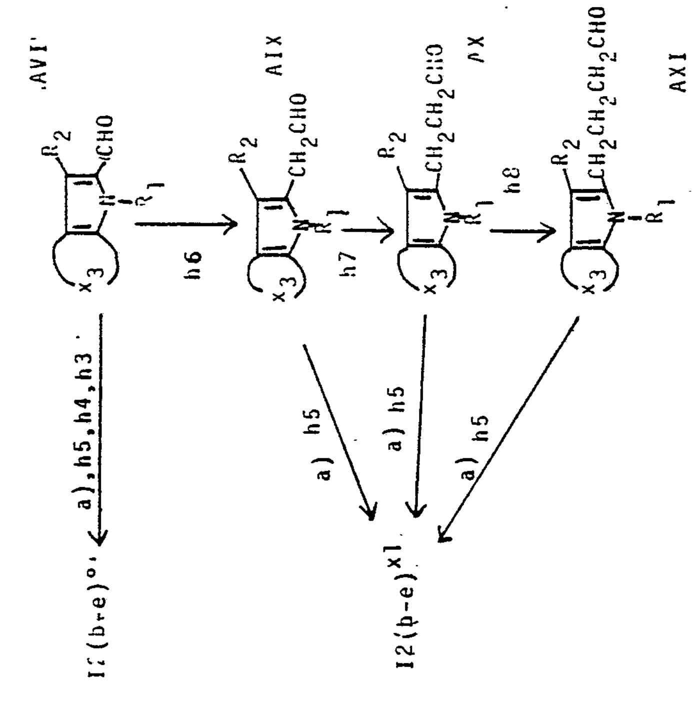


REACTION SCHEME 5

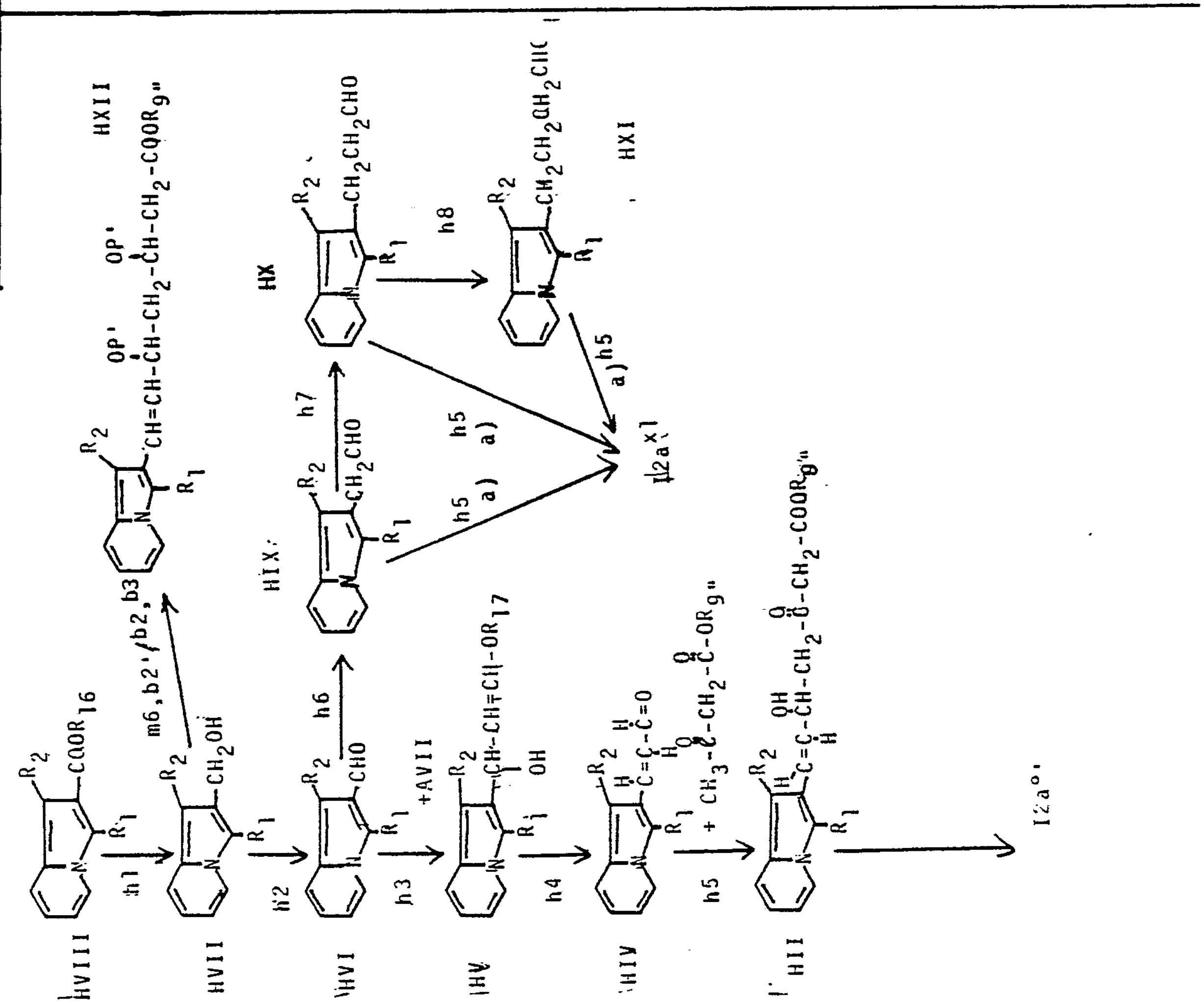

| REACTION STEP | TIME/STEP | SPECIAL CONDITIONS/REAGENTS | TEMPERATURES | ATMOS | SOLVENTS |
|---|---|---|---|---|---|
| a1 | Addition | 1. $BrCH=CH-OR^{12}$ + AK eg t-butyl Lithium → AVII<br>2. AVI + AVII → [ ]<br>3. [ ] quench eg sat. aq. $NH_4Cl$ - AV | 1. -40° to - 100° eg - 70°<br>2. As 1. | Anhydr. | 1. cyclic ether eg THF<br>2. As 1. |
| a2 | Cleavage | 1. AV+acid eg PTS + $H_2O$ → [ ]<br>2. [ ]+quench eg 10% aq $NaHCO_3$ → AIV | 1. 0° to 80° eg RT<br>2. As 1. | - | 1. aq cyclic ether eg 90% aqueous THF |
| a3 | Addition | 1. $CH_3COCH_2COOR_9$" eg ethyl acetoacetate + AK eg LDA → AIII<br>2. AIII + AIV → [ ]<br>3. [ ] + quench eg sat. aq. $NH_4Cl$ → AII | 1. -60° to -20° eg -40°<br>2. -60° to -20° eg -40°<br>3. -60° to RT | 1 Anhydr.<br>2. Anhydr. | 1. cyclic ether eg THF<br>2. As 1.<br>3. As 1. |
| b1 | Monobromination | BVII + brominating agent eg NBS → BV<br>-peroxidic catalyst benzoyl peroxide + active light | 60° to 100° eg 80° | Anhydr. | halogenated lower aliphatic hydrocarbon eg $CCl_4$ |
| b2 | Wittig reagent formn | BV + $P(OC_2H_5)_3$ → BIV | 100° to 140° eg 140° | Anhydr. | neat |
| b2' | | BV + $Ø_3P$ → BX | 60° to reflux preferably ≤ 150° | | Absolute ethanol |
| b3 | Wittig reacn | 1. BIII + BIV (or BX) → [ ]<br>2. [ ]+quench eg sat aq $NH_4Cl$ → BII | 1. -10° to -50° eg -30°<br>2.-50° to RT | Anhydr. | 1. cyclic ether eg THF<br>2. As 1. |

| | | | | | |
|---|---|---|---|---|---|
| c1 | Hydrazone formn | CVII + CVIII → CVI | Initiate 50° to 100° eg 80° to 90° then reflux | Anhydr. | neat - then toluene for azeotroping water. |
| c2 | Cyclisation | CVI → CV | 200° to 250° eg reflux | Anhydr. | inert high-boiling liq. solvent eg DEG ie 2-hydroxyethyl ether |
| c3 | N-substitution | 1. CV + AK eg NaH → Q<br>2. Q + CIV eg L = iodo → CIII | 1 = 2 : 200° to 250° eg reflux | Anhydr. | High-boiling liq. aromatic HC eg xylene. 1 = 2 |
| c4 | Dibromination | CIII + brominating agent eg. NBS → CII For 1.4 and 1.6 azaindoles alternatively use molecular bromine in sodium acetate containing acetic acid | ← as b1 →<br>90° to 95° | as b1 | → |
| c5 | hydrolysis | CII + $H_2O$ + acid acceptor eg $CaCO_3$ → AVIc | 40° to 120° eg reflux | - | aq. lower alcohol eg 70% aq. ethanol |
| d1 | hydrazone formn | DVII + CVII → DV | ← | as c1 | → |
| d2 | cyclisation | DV → DIV + DVI | ← | as c2 | → |
| d3 | methylation | 1. DIV + AK eg NaH → Q<br>2. Q + DIII eg $CH_3I$ → DII | ← | as c3 | → |
| d4 | formylation | 1. DII + AK eg N-Bu-Li → Q<br>2. Q + $R^{14}$-OOCH → [ ]<br>3. [ ] + quench eg sat aq. $NH_4Cl$ → AVI | 1. -30° to -60° eg -40° to -50°<br>2. as 1. them warm to RT<br>3. 20° to 40° eg RT | Anhydr. | 1 = 2 : cyclic ether eg THF |
| e1 | Acylation | CV + EIV → EIII when EIV is acylhalide, acid acceptor eg triethylamine used. | 20° to 100° eg. 100° when EIV is anhydride | Anhydr. | none needed when excess EIV serves as medium. |

| | | | | | |
|---|---|---|---|---|---|
| e2 | Tebbe reaction | 1. EIII + Tebbe reagents → [ ] in presence of pyridine<br>2. [ ] + methanol → EII | 1. -40° then RT<br>2. 0° then RT | Anhydr. | 1. toluene/ THF eg 20:1<br>2. toluene/ THF + diethyl ether |
| e3 | | EII + [H] → CIIIe<br>- under hydrogen pressure eg 40-50 psi + hydrogenation catalyst eg 5% palladium on C. | 20° to 60° eg RT | | lower alcohol eg ethanol |
| f1 | Grignard reagent formn | conventional manner | for preparation and utilisation of Grignard reagents | | |
| f2 | Grignard | | | | |
| f3 | hydrazone formn | FVIII + $R_2-CH_2-\overset{O}{\overset{\|}{C}}-\overset{O}{\overset{\|}{C}}-OEt$ → FVII | as c1 | | |
| f4 | cyclisation | FVII → FVI | as c2 | | |
| f5 | Reduction | FVI → FV conventional manner eg | lithium aluminium hydride in tetrahydrofuran | | |
| f6 | Protection | FV → FIV conventional manner eg | trimethylsilyl chloride | | |
| f7 | | FIV → FIII | as e1, e2, e3 | | |
| f8 | Deprotection | FIII → FII<br>+ tetrabutyl ammonium fluoride/glacial acetic acid | 0° to 50° eg RT | Anhydr. | cyclic ether eg THF |
| f9 | oxidation | FII → AVI conventional manner eg | manganese dioxide in toluene | | |
| g1 | hydrogenation | AII → GII<br>hydrogen pressure eg 40 to 50 psi + hydrogenation catalyst, eg 5% palladium on charcoal, 5% rodium on charcoal | 20° to 40° eg RT | | lower alcohol eg ethanol |

| | | | | | |
|---|---|---|---|---|---|
| h1 | Reduction | 1. HVIII + LAH → [ ]<br>2. Ethyl acetate + [ ] + quench with eq. ice-water → HVII | 1. -5° to 30° eg add LAH at 0° to raise to RT<br>2. Ice-water bath | | THF |
| h2 | Oxidation | HVII + $MnO_2$ → HVI | 20° to 140° eq. reflux | anhydr. | Inert hydrocarbon eq. toluene |
| h3 | Addition | HVI + AVII → HV | | as a1 | |
| h4 | cleavage | HV + acid eg PTS + $H_2O$ → HIV | | as a2 | |
| h5 | Addition | 1. form , AIII as step 1 in a3<br>2. HIV + AIII → [ ]<br>3. [ ] + quench aq $NH_4Cl$ → HII | 2. -80° to -20° eg -75° to -60°<br>3. 0° to RT | as a3<br>as a3 | |
| h6<br>h7<br>h8 | Wittig | 1. $(C_6H_5)_3P^{+}-CH_2OCH_3Cl^{-}$ + strong base eg NaH, phenyllithium or pref. n-Bu-Li<br>2. Add HVI (or AVI, AIX, AX, HIX, HX)<br>3. hydrolyse with strong acid eg. 70% perchloric in large molar excess | 1. -40° to 0°C pref. -35° to -20°C<br>2. -30° to 0° pref. 20° to 0°C.<br>0° to 30°C. | inert<br>inert | 1. Anhydrons inert organic solvent pref. ether solvent eg THF<br>2. As 1.<br>aqueous acid + ether solvent eg. THF |
| k1 | Nucleophinic Substitution | K + $Na_2S$ $9H_2O$ → KI mol ratio 2:1 | RT → reflux | | $CH_3OH$, EtOH ; PrOH pref. EtOH |
| k2 | Gassman reaction | t-butyl hypochlorite to KI . Add Na methoxide (1:1:1) | -65° to 40° | inert | $CH_2Cl_2$, $CHCl_3$ or $CCl_4$ pref. $CH_2Cl_2$ |
| k3 | Hydrogenolysis | KII + Excess Raney Nickel → KIII | 22° to 40° | inert | $CH_3OH$ EtOH pref EtOH |
| k4 | Acetylation | 1. $CH_3COCl$ ratio 1:1 or<br>2. pref. excess $(CH_3CO)_2O$ | 22° to 40° | | $CH_2Cl$, $CHCl_3$, $CCl_4$ toluene |
| k5 | Cyclisation | 1. Excess potassium-t-butoxide or<br>2. pref. $P_2O_5$ mol ratio 2:1 → KV | 250° to 300°<br>286° | | Dowtherm or other inert solvent |

| | | | | |
|---|---|---|---|---|
| m1 | 1. DVI + n-butyl lithium<br>2. Add benzene-sulfonyl chloride | 1. -80° to -50° pref. -78°<br>2. " " | inert<br>inert | 1. THF<br>2. THF |
| m2 | 1. L + n-Bu-Li<br>2. Add excess ethyl formate | 2. -50° to -40°C | inert<br>inert | 1. THF |
| m3 | Add to LI $K_2CO_3$+MeOH | reflux | inert | |
| m4 | 1. LII + 18-Crown- 6 + Excess $K^{+-}$O-t-Bu<br>2. add 1:1 equivalent of alkylating agent eg propyl-iodide | | inert<br>inert | $Et_2O$ |
| m5 | LIII + $NaBH_4$ | 20° to 25°C | inert | ethanol/diethyl ether |
| m6 | LIV + NBS/dimethyl sulphide → BV | -20° to 0°C | inert | $CH_2Cl_2$ |

The conditions given hereinabove are largely conventional for such reactions and can be varied in a conventional manner according to the particular intermediates/end products. This applies eg to molar ratios, temperature, reaction times and the like which are chosen according to principles well established in the art on the basis of reactants and conditions employed.

Reagents and starting materials described herein, e.g. compounds BVII, CV, CVI, CVII, CVIII, DIV, DV, DVI, and DVII are known and obtainable by known means, or where not known, may be obtained by adaptation of methods reported in the literature for the preparation of known analogs; some compounds being commercially available.

For example, compounds BVII, CV, DIV, FVI and KV, and their preparation is described in Canadian Journal of Chemistry 44, 2455 (1966).

Journal of the Chemical Society 1970, 303 and Belgian Patent 840,362.

The final products and intermediate compounds described herein may be recovered and refined, where such is desired, by conventional means, such as by crystallization, distillation or chromatographic techniques, such as column or thin layer chromatography, e.g. silica gel column chromatography.

Reagents and reaction products which are mixtures of stereoisomers (cis, trans and optical) can be separated by conventional means at whatever stage of synthesis is appropriate. Such methods include recrystallization, chromatography, eg HPLC, formation of esters with optically pure acids and alcohols or of amides and salts with subsequent reconversion with retention of optical purity. For example diastereoisomeric (-)-α-naphthylphenylmethylsilyl derivatives of a lactone type end product of formula I may be separated by conventional means, eg as disclosed in USP 4,613,610.

In the Wittig reaction (process b3), if in place of a Compound BIII there is employed a 4R, 6S-pyranyl-aldehyde of the formula BIIIa or BIIIb:

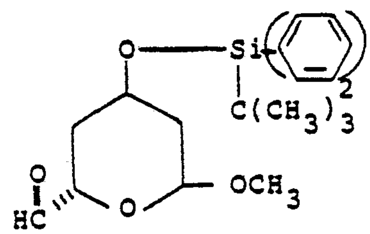

BIIIa

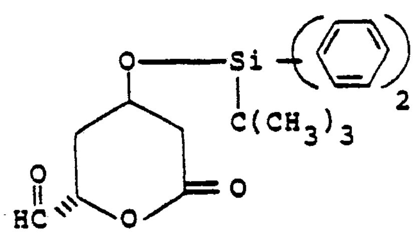

BIIIb ,

corresponding substituted-pyranyl analogs of compounds BII are obtained which can be converted to corresponding lactone final products (I4°) having the 4R,6S form which can be converted to corresponding Compounds I wherein Z = a), eg I2°, having the 3R,5S form. Compounds BIIIa and BIIIb are known and their use in producing olefinic lactones is disclosed in the literature, eg USP 4,474,971 and World Patent Application WO86/00307 (corresponding to USP 4,613,610 (issuing Sept. 23, 1986).

Intermediate derivatives wherein A = a) and Z = b) obtained by use of Compounds BIIIa or BIIIb can be converted to corresponding compounds in which A = $-CH_2-CH_2-$and Z = b) by hydrogenating under the conditions of process g1).

Compounds I having one or more of the following characteristics are preferred:

a) A = (E)-CH=CH-

b) Z = a)

c) when Z = a, $R_8$ = M, especially sodium.

d) when one Y = N it is preferably $Y^4$

e) and when A = a) and Z = a) the optically active isomer of the 3R,5S form.

The compounds of Formula I possess pharmacological activity in particular as competitive inhibitors of 3-hydroxy-3-methylglutaryl coenzyme A (HMG-CoA) reductase and as a consequence are inhibitors of cholesterol biosynthesis as demonstrated in the following tests:

Test A: In Vitro Microsomal Assay of HMG-CoA Reductase Inhibition:

As described in EP 114027 or 117228 (dosage range 0.0005 to 2,000 µm).

Test B: In Vivo Cholesterol Biosynthesis Inhibition Test:

As described in EP 114027 or 117228 (dosage range 0.01 to 200mg/kg).

The compounds are thus indicated for use as hypolipoproteinemic and anti-atherosclerotic agents.

As indicated suitable daily dosage for use in the treatment of hyperlipoproteinemia and atherosclerosis is from about 0.1 to 2,000 mg preferably 1 to 140 mg suitably administered either once a day or in divided dosages two to four times daily. A typical dosage unit for oral administration may contain 0.025 to 500 mg, preferably 0.25 to 500 mg especially 0.25 to 70 mg.

The invention therefore also concerns a method of treating hyperlipoproteinemia or atherosclerosis by administration of a compound of formula I in free acid form or in the form of a physiologically -acceptable ester or lactone thereof or in a pharmaceutically acceptable salt form as well as compounds for use as pharmaceuticals eg as hypolipoproteinemic and anti-atherosclerotic agents.

The compounds may be administered alone, or in admixture with a pharmaceutically acceptable diluent or carrier, and, optionally other excipients, and administered orally in such forms as tablets, or capsules or parenterally in such forms as injectible solutions or suspensions.

The preferred compositions from the standpoint of ease of preparation and administration are solid compositions, particularly tablets and hard-filled or liquid-filled capsules.

Such compositions also form part of the invention.

The following examples in which all temperatures are in °C illustrate the invention.

EXAMPLE I

Ethyl (E)-7[3-(4-fluorophenyl)-l-isopropyl-lH-pyrrolo-[2,3-b] pyridin-2-yl]-3,5-dihydroxyhept-6-enoate (eyrthro)A compound Ie in which $R_1$ = isopropyl, $R_2$ = 4-fluorophenyl, A = (E)-CH=CH-and Z = a)

Step l:l-(4-fluorophenyl-2-propanone-2-pyridinyl hydrazone

In a vessel fitted with a condenser under nitrogen, 2l.8g (0.2M) of 2-hydrazino pyridine and 30.4g (0.2M) of 4-fluorophenyl acetone are heated at 80-90° for about 30 to 60 mins. Toluene is added and the mixture refluxed until all water is azeotropically distilled off. The mixture is then evaporated to dryness yielding crude product of this step as a yellow gum. The gum is distilled under reduced pressure (l50-l60° at about 50 microns) to obtain the product of this step (as a yellow gum).

Step 2:3-(4-fluorophenyl)-2-methyl-lH-pyrrolo [2,3-b] pyridine

Under nitrogen, 33g (0.l36M) of the hydrazone product of Step l, above, in 300ml of diethylene glycol is refluxed (about 250°) for 7 to 8 hours, and then quenched with water. Solids precipate and are recovered by filtration. The solids are washed with petroleum ether and taken to nearly dryness by evaporation, yielding light brown solids. The solids are treated with a large volume of ethyl acetate. Some solids remain which are collected by filtration and dried to yield slightly gray solids. (mp 224 to 227°). The filtrate is dried and then treated with charcoal and concentrated until precipitation begins. The solids are collected by filtration, washed with ether/petroleum ether (50/50) yielding yellow solids (mp 222 to 226°). The crops of solids are combined to give the title product of this step, for use in Step 3, below. This product may also be called 3-(4-fluorophenyl)-2-methyl-7-azaindole.

Step 3: I-acetyl-3-(4-fluorophenyl)-2-methyl-IH-pyrrolo [2,3-b] pyridine

Under nitrogen, 4.I5g (0.0I84M) of the azaindole product of Step 2, above, and I00ml of acetic acid anhydride are heated at about I00° for about 2 hours. The mixture is cooled and then evaporated to dryness to obtain a residue. The residue is triturated with ether to give cream-coloured solids (mp 84 to 87°) which are recovered by filtration. The filtrate is evaporated to dryness, to give a residue which is dissolved in methylene chloride, filtered through silica and concentrated to yield tan sticky solids, which after trituration with ether/petroleum ether (I:I) yield cream coloured solids (mp 84 to 86°). Both crops of solids are combined for use in Step 4, below.

Step 4: I-(I-methyl-eth -I-enyl)-3-(4-fluorophenyl)-2-methyl-IH-pyrrolo[2,3-b]pyridine

Under nitrogen, a solution of 0.678g (0.00253M) of the acetyl product of Step 3, above, 20ml of absolute toluene (dried over molecular sieves), Iml of absolute THF (distilled over LAH) and 2 drops of absolute pyridine is cooled to about -40° and to it is slowly added 5.Iml of Tebbe Reagent. The resulting mixture is held at about -40° for one half hour, then permitted to warm to RT (over 3/4 hour). 20ml of ether is then added and the mixture cooled to about 0°. Iml of methanol is then added to the mixture dropwise. The resulting mixture is then allowed to stand at RT for about 64 hours.

The mixture is then filtered through celite, then filtered through alumina (Woelm B, Act.I), using ethyl acetate as wash.

The mixture is then evaporated to dryness to yield an orange gum, which is refined by chromatographing on prep. plates developed with ether/petroleum ether (40/60). The main band is eluted with ethyl acetate to give the title product of this step, as slightly yellow solids, mp 84 to 87°.

Step 5: I-isopropyl-2-methyl-3-(4-fluorophenyl)-IH-pyrrolo [2,3-b]pyridine

Into a parr vessel are placed 330 mg of the olefinic product of Step 4, above, 20ml of I00% ethanol and I00 mg of 5% palladium on carbon. Hydrogen gas is added at 40 to 50 psi and held at RT for about I6 hours. Ethyl acetate is then added, the mixture filtered through celite and then evaporated to dryness to give the title product of this step as a slightly yellowish gum, which solidified on standing to a light cream solid (mp 57 to 68°).

Step 6: I-isopropyl-2-bromomethyl-3-(4-fluorophenyl)-IH-pyrrolo[2,3-b]pyridine

Under nitrogen, is mixed 560mg (2.09mM) of the N-isopropyl product of Step 5, above, 40ml of carbontetrachloride (dried over molecular sieves), 376mg (2.09mM) of N-bromosuccinimide (recrystallized) and II7.4mg (0.46mM) of benzoyl peroxide. The mixture is heated at about 80° for I hour under a standard I50 watt incandescent lamp.

The mixture is cooled to RT, then ether added, and the mixture filtered through a pad of silica gel. The filtrate is then evaporated to dryness to obtain the product of this step as yellow gummy solids which are refrigerated under $N_2$, until used in Step 7, below.

Step 7: diethyl [[3-(4-fluorophenyl)-I-isopropyl-IH-pyrrolo [2,3-b]pyridin-2-yl]methyl]phosphonate

Under nitrogen, 0.95g of the bromo-product of Step 6, above, in I0ml of triethyl phosphite is heated at about I40° for about 4 hours.

The mixture is then cooled and evaporated to dryness to yield I.373mg of crude product which is refined by chromatography (prep plates), developed with petroleum ether/ether (60/40) yielding a main band which is then eluted with ethyl acetate to obtain the title product of this step as a yellow gum.

Step 8: ethyl (E)-7 [3-(4-fluorophenyl)-l-isopropyl-lH-pyrrolo[2,3-b]pyridin-2-yl]-(3,5-di(diphenyl-t-butylsiloxy)-)-hept-6-enoate (erythro)

Under nitrogen, 232mg (0.574mM) of the phosphonate reagent of Step 7, above, in l2ml of absolute THF is cooled to about -20° to -30° and there is added thereto 0.362ml (0.688mM) of l.9M LDA (in cyclohexane), and the mixture stirred for one half hour. There is then added 470.5mg (0.742mM) of ethyl erythro-3,5-di(diphenyl-t-butylsiloxy)-6-oxo-hexanoate in l5ml of absolute THF. The mixture is stirred for about 45 mins at about -30°.

The mixture is then quenched with saturated aq. ammonium chloride, extracted with ethyl acetate, the extract dried, filtered through a pad of silica gel, and evaporated to dryness to obtain crude title product of this step as a residue (orange gum). The residue is refined by chromatography (prep plates) developed with petroleum ether/ether (60/40). The second of the 3 main bands (the largest) is eluted with ethyl acetate to yield the title product of this step as a light greenish-yellow gum.

Step 9: ethyl (E)-7 [3-(4-fluorophenyl)-l-isopropyl-lH-pyrrolo[2,3-b]pyridin-2-yl]-3,5-dihydroxy-hept-6-enoate (erythro)

Under nitrogen, a mixture of 0.575g (about 0.628mM) of the protected product of Step 8, above, in 30ml of absolute THF (distilled over LAH), 2.5ml (2.5mM) of tetrabutylammonium fluoride (lM in THF) and 0.l4ml (2.5mM) of glacial acetic acid is stirred at RT for about l8 hours. The mixture is taken to dryness to yield crude title product of this example as a dark gum, which is refined by chromatography (prep plates), developed with methylene chloride/methanol (95:5). The main band is eluted with ethyl acetate to yield the title product of this example as light yellow solids mp l20 to l23° (about 97% erythro)

## EXAMPLE 2

Ethyl (E)-7-[3-(4-fluorophenyl)-l-methyl-lH-pyrrolo[2,3-b]pyridin-2-yl]-3,5-dihydroxy-hept-6-enoate (erythro)A compound le in which $R_1$ = methyl, $R_2$ = 4-fluorophenyl, A = (E)-CH=CH-and Z = a)

Step l: l-(4-fluorophenyl)-2-ethanone, 2-pyridylhydrazone

In a vessel, equipped with a Dean-Stark trap, under an atmosphere of nitrogen gas, approximately l6g (0.ll6M) of 4-fluorophenylacetaldehyde and l2.6g (0.ll6M) of 2-hydrazinopyridine are heated to about 80° to 90° for from 30 to 60 minutes. Toluene is then added and the reaction mixture refluxed with a Dean-Stark trap until all water is removed (about 4 hours). This results in a yellow-orange solution which is evaporated (under vacuum) to dryness, yielding the title product of this step as an orange viscous liquid.

Step 2: 3-(4-fluorophenyl)-lH-pyrrolo[2,3-b]pyridine

In a vessel, under nitrogen, about 27g of the crude product of Step l, above, in about 300ml of diethylene glycol is refluxed (at about 250°) for about 7 hours. The contents are cooled, and then quenched with water yielding a yellow precipitate. The precipitate is washed thoroughly with water, then once with petroleum ether. The product is dissolved in ethyl acetate, dried and then concentrated by evaporation until precipitation begins. The precipitate is recovered by filtration, and washed first with cold ethyl acetate, then with petroleum ether yielding the title product of this step as yellow solids (mp l90° to l92°).

Step 3: l-methyl-3-(4-fluorophenyl)-lH-pyrrolo[2,3-b]pyridine

Into a vessel under nitrogen is added 0.48g (l0mM) of a 50% dispersion of sodium hydride (in paraffin), 40ml of absolute xylene (dried over molecular sieves), and l.94g (l0mM) of the azaindole product of Step 2, above. The mixture is refluxed for 3 hours, then allowed to cool to room temperature. Then there is added slowly 2.82g (20mM) of methyl iodide in l0ml of absolute xylene (dried over molecular sieves), and the mixture refluxed for about 2 hours.

Ethanol is then cautiously added to the reaction mixture (to decompose unreacted hydride). The mixture is then cooled, and the xylene solution is extracted with 2N hydrochloric acid (some suspension occurs which is eliminated by filtering). The aqueous acid (containing the reaction product) is made basic and the product extracted with ethyl acetate. The ethyl acetate extract is dried and then evaporated to dryness to obtain a brown gum. The gum is dissolved in methylene chloride to which is added a small amount of anhydrous sodium sulfate and charcoal and then filtered through silica gel, eluting with methylene chloride, to obtain the title product of this step as light tan solids (mp 95-98°).

Step 4: l-methyl-2-formyl-3-(4-fluorophenyl)-lH-pyrrolo[2,3-b]pyridine

To a vessel, under nitrogen, is charged l.05g (4.65mM) of the product of Step 3, above, in 65ml of absolute THF (distilled over lithium aluminium hydride), and the solution cooled to about -40° to -50°. 3.53ml (5.6lmM) of n-butyl-lithium (l.6 molar in hexane) is added slowly to the reaction mixture at about -40° to -50° and the mixture held at that temperature for l hour. (A reddish colour develops). 3.2ml of dry ethyl formate (97%) (dried over molecular sieves) is added at about -40° to -50°, and the mixture stirred for 2 hours; the colour of which lightens to yellow. The mixture is then allowed to warm to RT and held for about l6 hours. Saturated aqueous ammonium chloride is then added to quench the reaction mixture, which is then extracted with ethyl acetate. The extract is dried, and then evaporated to dryness to obtain a residue. The residue is refined by chromatography (prep plates) and developed with methylene chloride. The fastest moving heavy band contains the product of this step and is recovered as yellow solids by eluting with ethyl acetate (the second band contains starting material).

Step 5: 3-[l-methyl-3-(4-fluorophenyl)]pyrrolo[2,3-b]pyridin-2-yl]-prop-2-enal

Under a nitrogen atmosphere, 0.302mg (2mM) of cis-l-bromo-2-ethoxyethylene (purity 95%) in l5ml of absolute THF (distilled over LAH) is cooled to about -70° and added thereto is 2.35ml (4mM) of t-butyl-lithium (l.7M in pentane). The mixture is stirred at about -70° for 2 hours. 254mg (lmM) of the product of Step 4, above, in l0ml of absolute THF (distilled over LAH) is then slowly added. The mixture is stirred at about -70° for 2 to 3 hours. Then while cooling quenched with saturated aqueous ammonium chloride. Water is added and the mixture extracted with ethyl acetate. The extract is dried and then evaporated to dryness to give intermediate product as a yellow gum, which is charged to a mixture of about 50ml of 90% aqueous THF and 75mg of p-toluene sulfonic acid monohydrate.

The mixture is stirred for about l6 hours resulting in a solution, which is then quenched with l0% aqueous sodium bicarbonate, and extracted with ethyl acetate. The extract is dried, and then evaporated to dryness to give a yellow gummy foam, which is then chromatographed on prep plates developed with petroleum ether/ether (50/50). The main band is eluted with ethyl acetate, the crude material recovered, and upon trituration with petroleum ether/ether (50/50) gives the title product of this step as yellow solids mp l55° to l60°.

Step 6: ethyl (E)-7[3-(4-fluorophenyl)-l-methyl-lH-pyrrolo [2,3-b]pyridin-2-yl]-5-hydroxy-3-oxo-hept-6-enoate

A dianion reagent (a AIII) is prepared by mixing, under nitrogen, l5ml of absolute THF (distilled over LAH) 3.6ml (25.36mM) of diisopropylamine (distilled over LAH). The mixture is cooled to 0° and there is added l6.2ml (25.92mM) of l.6M n-butyl-lithium (in hexane). After l5 minutes, the mixture is cooled to -30° to -40°, and there is added l.5ml (l2mM) of ethyl acetoacetate (distilled), and the mixture stirred at -30° to -40° for l hour.

l0ml of the dianion mixture is added, at -30° to -40° to 0.lg (0.357mM) of the olefinic aldehyde of Step 5, above, in l0ml of THF (distilled over LAH) and the mixture stirred at -30° to -40° for 2 hours.

The reaction mixture is quenched with saturated aqueous ammonium chloride (while still cooling), then water added and extracted with ethyl acetate. The ethyl acetate solution is dried and then taken to dryness to give a yellow orange viscous liquid which is chromatographed on prep plates developed with methylene chloride/methanol (98/2), and elution of the major band with ethyl acetate to provide the title product of this step as a yellow gum.

Step 7: ethyl (E)-7[3-(4-fluorophenyl)-l-methyl-lH-pyrrolo [2,3-b]pyridin-2-yl]-3,5-dihydroxy-hept-6-enoate

Under nitrogen, 0.ll7g (0.285mM) of the keto-hydroxyester product of Step 6, above, is mixed with 20ml of absolute THF (distilled over LAH), 5ml of methanol and 0.37lml (0.37lmM) of lM triethyl borane (in THF). 2.3ml of air is slowly bubbled into the mixture at RT, then stirred for 2 hour hours.

The mixture is then cooled to below -75° and in one portion is added l4.7mg (0.388mM) of sodium borohydride, and the resulting mixture stirred at below -75° for 3 hours.

To the still cooled mixture is then added 0.2ml of acetic acid, and the mixture allowed to warm slowly to RT over about l6 hours.

The mixture is then slightly cooled and there is added 4ml of l0% aqueous sodium bicarbonate, and the mixture extracted with ethyl acetate. The extract is dried and evaporated to obtain a residue. To the residue is added l0ml of methanol, and the mixture stirred at RT. The mixture is then refluxed for 2 hours, and then separated by chromatography, (prep plates developed using methylene chloride/methanol (95:5)). The second major band is eluted using ethyl acetate, and on removing solvent, yields the title product of this example, as a yellow gum.

EXAMPLE 3

(E)-7-[3-(4-fluorophenyl)-l-isopropyl-lH-pyrrolo[3,2-b]pyridin-2-yl]-3,5-dihydroxy-hept-6-enoic acid, l,l-dimethylethyl ester [R-(R*S*)]A compound lb in which $R_1$ = isopropyl, $R_2$ = 4-fluorophenyl, A = (E)-CH=CH-and Z = a)

Step l: l,l'-[thiobis(methylene)]bis[4-fluorobenzene]

500ml absolute ethanol, 4lgm (0.l72M) sodium sulfide nonahydrate and 50gm (0.35M) 4-fluorobenzyl chloride are heated slowly to reflux and refluxed for l6 hours. The mixture is then cooled, added to water and extracted with ethyl acetate. The extract is evaporated to dryness over anhydrous sodium sulphate to yield the title product as an oil.

Step 2: 2-[(4-fluorophenyl)(4-fluorophenylmethylthio)]-methyl-3-pyridinamine

l.2g (0.0l3M) of 3-amino-pyridine and 55ml of dry methylene chloride are cooled to -65°. l.4g (0.0l3M) t-butyl hypochlorite is added dropwise over 5 minutes and the mixture stirred for 25 minutes, maintaining the temperature at -65°. Dropwise over 5 minutes is added 3.2g of the product of Step l above, with stirring for 40 minutes, until the reaction mixture turns dark brown. Added is a solution of l.4g (0.026M)sodium methoxide in 9ml methanol. The temperature is maintained at -65° for l hour, and then the mixture is heated to 40° and maintained for l6 hours. The reaction mixture is cooled and added to cold water, before extraction with methylene chloride, drying and reduction to a crude brown oil. The crude product is then column chromatographed on silica gel to obtain the title product (mp ll4° to ll6.5°).

Step 3: 2-(4-fluorophenyl)-methyl-3-pyridinamine

Under dry nitrogen l7g of the product of Step 2, above, and 500ml of absolute ethanol are sweep heated over 30 minutes to 40°. The mixture is cooled to RT, 22g activated Raney Nickel is added and heated slowly to 40°. After 4 hours the mixture is filtered through celite and the volume reduced to dryness to produce the title product as a crude brown oil.

Step 4: N-[2-(4-fluorophenyl)-methyl-pyridin-3-yl]acetamide

l0.44g of the product of Step 3, above, and 40ml of acetic anhydride are warmed slowly to 40° for 2 hours. The mixture is added to water, extracted with ethyl acetate, dried and reduced in volume. Petroleum ether is added to achieve the title product (mp l65° to l67°).

Step 5: 3-(4-fluorophenyl)-2-methyl-lH-pyrrolo[3,2-b]pyridine

lg of the product of Step 4, above, 60ml Dowtherm and l.2g of phosphorus pentoxide are heated to reflux for 45 minutes. The mixture is cooled to RT, 27ml of methanol added and stirred for l hour. 60ml of 2N hydrochloric acid is added, the mixture stirred for l hour and extracted with ether. The extract is filtered, the aqueous layer acidified, ice added and then 2N sodium hydroxide is added until the mixture is basic. After methylene chloride extraction, drying, evaporation to dryness and chromatography, the title product is produced (mp l99° to 206°).

If the product of Step 5, above, is taken through steps analagous to those of Example l, Steps 3 to 7, the following products may be obtained:

Step 6: l-[3-(4-fluorophenyl)-2-methyl-lH-pyrrolo[3,2-b] pyridin-l-yl]ethanone (mp l22° to l27°)

Step 7: 3-(4-fluorophenyl)-2-methyl-l-(l-methylethenyl)-lH-pyrrolo[3,2-b]pyridine (thick yellow oil)

Step 8: 3-(4-fluorophenyl)-2-methyl-l-isopropyl-lH-pyrrolo [3,2-b]pyridine (mp l53° to l57°)

Step 9: 2-bromomethyl-3-(4-fluorophenyl)-l-isopropyl-lH-pyrrolo[3,2-b]pyridine

Step l0: [3-(4-fluorophenyl)-l-isopropyl-lH-pyrrolo[3,2-b] pyridin-2-yl]methyl-diethyl ester phosphoric acid

Step ll: (E)-7-[3-(4-fluorophenyl)-l-isopropyl-lH-pyrrolo[3,2-b]pyridin-2-yl]-3,5-di[(l,l-dimethylethyl) diphenylsilyloxy]-hept-6-enoic acid l,l-dimethylethyl ester [R-(R*S*)]

To l3mg (0.l3M) of diisopropylamine and 2ml of dry THF is added 0.08ml (0.l3mM) of n-butyl lithium at 0-5°. After l5 minutes, 49mg (0.l2mM) of the product of Step l0, above, in dry THF is added at -35°, stirred for 2 hours and the temperature allowed to rise to -l0°. The mixture is cooled to -30° and l04mg (0.l5mM) of [R-(R*S*)] 3,5-di[(l,l-dimethylethyl)diphenylsilyloxy]-6-oxo-hexanoic acid-l,l-dimethylethylester in a minimum volume of THF. The reaction mixture is stirred for l6 hours and the temperature allowed to rise to RT before being added to saturated aqueous ammonium chloride and extracted with ethyl acetate. The organic layers are dried, reduced to dryness, and chromatographed to obtain the title product as a thick yellow oil.

Step l2: (E)-7-[3-(4-fluorophenyl)-l-isopropyl-lH-pyrrolo [3,2-b]pyridin-2-yl]-3,5-dihydroxy-hept-6-enoic acid l,l-dimethylethyl ester [R-(R*S*)]

Using the product of Step ll, above, and employing methods analagous to those of Example l, Step 9, above, the title product of this example may be obtained. (mp 62°-65°)

EXAMPLE 4

(E)-7-[3-(4-fluorophenyl)-l-isopropyl-lH-pyrrolo[3,2-b] pyridin-2-yl]-3,5-dihydroxy-hept-6-enoic acid, l,l-dimethylethyl ester hydrochloride [R-(R*S*)]A compound Ib in which $R_1$ = isopropyl, $R_2$ = 4-fluorophenyl, A = (E)-CH=CH-and Z = a)

To 4ml of absolute ether is added 25mg (0.053mM) of the product of Example 3, above, at RT. To this mixture is added l90mg (5.3mM) of hydrogen chloride gas. Filtering produces the title product of this example as a light yellow crystalline solid (mp ll8°to l22°).

Repeating the procedure of this example, but using as starting material the product of Example l, above, and likewise with the product of Example 2, above, there may be obtained the corresponding hydrochloride salts thereof.

EXAMPLE 5

Sodium (E)-7-[3-(4-fluorophenyl)-l-methyl-lH-pyrrolo[2,3-b] pyridin-2-yl]-3,5-dihydroxy-hept-6-enoateA compound Ie in which $R_1$ = methyl, $R_2$ = 4-fluorophenyl, A = (E)-CH=CH-and Z = a)

A) To a solution of 43mg (0.l04mM) of the product of Example 2, above, in l5ml of ethanol, is added, at RT, 3ml of water and 0.099ml (0.099mM) of lN aqueous sodium hydroxide, and the mixture stirred for l hour at RT.

The mixture is evaporated to dryness to obtain a residue, which is dissolved in chloroform, then evaporated to dryness again. The residue is then dissolved in chloroform, dried and evaporated to dryness to give the product of this example, as light yellow-orange sticky solids.

B) Repeating the procedure of this example, but using as starting material the product of Example l, above, and likewise with the product of Example 3, above, there is accordingly obtained the corresponding sodium salt thereof.

## EXAMPLE 6

Ethyl (E)-7-[3-(4-fluorophenyl)-l-isopropyl-lH-pyrrolo[2,3-b] pyridin-2-yl]-3,5-dihydroxy-hept-6-enoate (erythro)

Step l: l-isopropyl-2-dibromomethyl-3-(4-fluorophenyl)-lH-pyrrolo[2,3-b]pyridine

Under nitrogen, 0.54g (2mM) of l-isopropyl-2-methyl-3-(4-fluorophenyl)-lH-pyrrolo[2,3-b]pyridine (obtainable by Step 5 of Example l, above) in 40ml of carbon tetrachloride (dried over molecular sieves), l.28g (7.lmM) of N-bromosuccinimide (recrystallised) and 0.048g (o.2mM) of benzoyl peroxide are refluxed for 2 hours under a l50 Watt light. The mixture is then cooled, solids filtered off, and then evaporated to dryness to obtain the title product of this step as a yellow sticky solid which is used directly for the next step.

Step 2: l-isopropyl-2-formyl-3-(4-fluorophenyl)-lH-pyrrolo [2,3-b]pyridine

lg of the product of Step l, above, in 35ml of l00% ethanol is mixed with 0.4g of calcium carbonate (powdered) and l5ml of water. The mixture is refluxed for about 4 hours, then cooled, quenched with water and extracted with ethyl acetate. The extracts are dried, and then evaporated to dryness to yield a dark amber gum, which is chromatographed on prep plates using petroleum ether/ether (85/l5) and the largest (second of three) major band eluted with ethyl acetate to obtain the title product as yellow solids (mp 95° to l0l°).

Using the product of Step 2, above, and following the procedure of Steps 5 to 7 of Example 2, above, there is accordingly obtained the title product of this example. The

The corresponding sodium salt may be obtained by hydrolysis with eg sodium hydroxide.

## EXAMPLE 7

Ethyl (E)-7-[3-(4-fluorophenyl)-l-isopropyl-lH-pyrrolo[3,2-c] pyridin-2-yl]-3,5-dihydroxy-hept-6-enoateA compound Ic in which $R_1$ = isopropyl, $R_2$ = 4-fluorophenyl, A = (E)-CH=CH-and Z = a)

Step l: 4-hydrazinopyridine HCl

6.56g (0.0437M) of 4-chloropyridine HCl and 25ml of ice-cold sodium hydroxide (2N) are mixed and extracted with ether. The solution is dried over $Na_2SO_4$, filtered and evaporated to dryness to produce a light yellow oil. This is added to 7.5g (0.l5M) of $NH_2NH_2.H_2O$ in l5ml of absolute ethanol, and heated at l00° for l6 hours in a sealed vessel. After cooling, the mixture is evaporated to dryness, ethanol added and reevaporated to dryness to produce the title product as light green solids.

Step 2: l-(4-fluorophenyl)-2-ethanone, 4-pyridylhydrazone

l.455g (l0mM) of the title product of Step l, above, l.38g (l0mM) of p-fluorobenzaldehyde and 0.l64g (20mM) of anhydrous sodium acetate are heated at 80°-90° for 30 to 60 minutes. Toluene is added and water azeotropically distilled off for 4 hours. While still warm the insoluble solids are filtered off, washed with $CH_2Cl_2$ and dried. The filtrate is evaporated to dryness, the residue dissloved in $CH_2Cl_2$ and filtered through silica gel, using first $CH_2Cl_2$ and then $CH_2Cl_2/CH_3OH$ (95:5), to yield the title product as a brown gum.

Step 3: 3-(4-fluorophenyl)-lH-pyrrolo[3,2-c]pyridine

If the crude product of Step 2, above, is treated in a manner analagous to Example 2, Step 2, above, there is correspondingly obtained the title product of this step as a dark red gum.

Step 4: l-benzene sulfonyl-3-(4-fluorophenyl)-lH-pyrrolo [3,2-c] pyridine

l06mg (0.5mM) of the product of Step 3, above, in 5ml of absolute THF is cooled to -78°. 0.328ml (0.525mM) of l-6M n-butyl-lithium in hexane is added and the mixture stirred for l hour whilst allowing the temperature to rise to 0°. The mixture is then re-cooled to -60° and 97mg (0.55mM) of benzene sulfonyl chloride is added. After allowing to warm to RT overnight, the mixture is quenched with aqueous $NaHCO_3$ and extracted with ethyl acetate. After washing with $NaHCO_3$ the mixture is dried over $Na_2SO_4$ and evaporated to dryness. The residue is triturated with ether and the solids produced precipitated and dried to produce the title product of this step.

Step 5: l-benzene sulfonyl-2-formyl-3-(4-fluorophenyl)-lH-pyrrolo[3,2-c]pyridine

35.2mg (0.lmM) of the product of Step 4, above, in 4ml of absolute THF is cooled to -40° to -50°. To this is then slowly added 0.075ml (0.l2mM) of n-butyl-lithium (l.6M in hexane) and the mixture stirred for l hour at -40° to -50°. 0.lml (l.2mM) of ethyl formate is added with stirring for 2 hours at -40° to -50°. The mixture is quenched with aqueous ammonium chloride and extracted with ethyl acetate. The resulting solution is dried over $Na_2SO_4$ and evaporated to dryness. The resulting product is applied to prep plates in $CH_2Cl_2/CH_3OH$ (98:2) and the bands eluted with ethyl acetate to give the title product of this step as a yellow gum.

This product may be reacted according to reaction steps m3 through to b3 in Reaction Scheme 4, to produce the title product of this example. The corresponding sodium salt may be obtained by hydrolysis with eg sodium hydroxide.

EXAMPLE 8

Ethyl 7-[3-(4-fluorophenyl)-l-isopropyl-2-indolizinyl]-3,5-dihydroxy-hept-6-enoate (trans)A compound la in which $R_1$ = 4-fluorophenyl, $R_2$ = isopropyl, A = (E)-CH=CH-and Z = a)

Step l: 2-(4'-fluorobenzoyl)-3-(2-pyridinyl)propenoic acid methyl ester

30g (0.l5M) of methyl 4-fluorobenzoyl acetate are added to l6.05g (0.l5M) of 2-pyridine carboxaldehyde. 4 drops of piperidine are then added, and the mixture stirred at RT for about l6 hours. The mixture is then heated at about 80° for about 5 hours during which the mixture thickens markedly, yielding crude title product of this step. The mixture is then cooled and solidifies on standing. The solids are broken up, washed with methanol, then triturated with hot methanol and the solution evaporated to dryness, then crystallized from petroleum ether/methanol (50:50), yielding the refined title produce of this step for use in Step 2, below.

Step 2: 2-(4-fluorobenzoyl)-3-isopropyl-3-(2-pyridinyl) propanoic acid, methyl ester

l.89ml of 2M (0.0035M) isopropyl magnesium chloride (in diethyl ether) is added to 25ml dry THF. At 0°, 40mg of cuprous iodide (Cu(I)I) 0.l7mM is added and the mixture cooled to -70°. lg of the 2-propenoic acid ester of Step I in 8ml of dry THF is added rapidly. The temperature of the mixture is raised to -50°, and maintained and then maintained for one half hour at -50° to -70°. There is then added 40mg of cuprous iodide and the mixture is allowed to warm slowly to RT and stirred for about l6 hours.

Saturated aqueous ammonium chloride is then added to the mixture, and crude product of this step extracted by ethyl acetate. The extracts are combined and evaporated to obtain crude product which is used as such in the next step, (step 3).

Step 3: 3-(4-fluorophenyl)-l-isopropyl-2-indolizine-carboxylic acid, methyl ester

23g of the crude 2-pyridinepropanoic acid ester product of Step 2, above, is added to 300ml of acetic acid anhydride. The mixture is slowly heated to reflux and gently refluxed for 3.5 hours. The mixture is then added to ice-water and 0.5 liter of 2N NaOH and extracted with ethyl acetate. The organic phase is dried and evaporated to obtain crude title product of this step. The product is refined by chromatographing through silica gel (eluting with ethyl acetate/hexane (20:80)). The fractions are combined and product recovered by crystallising (mp l39° to l42°).

Step 4: 3-(4-fluorophenyl)-l-isopropyl-2-indolizine-methanol (may also be called 3-(4-fluorophenyl)-l-isopropyl-2-hydroxymethylindolizine)

5.lg (0.0l6M) of the indolizine product of Step 3, above, in 300ml of dry THF is cooled to 0°. l.8g of LAH is slowly added over a period of l hour. With stirring, the temperature of the mixture is then cooled (in an ice-water bath) and ethyl acetate added dropwise to decompose unreacted LAH. Ice-water is then slowly added, and the product extracted with ethyl acetate. The extracts are combined, dried and evaporated to dryness to obtain the title product of this step (as a semi-solid).

Step 5: 3-(4-fluorophenyl)-l-isopropyl-2-indolizine-carboxyaldehyde

4.7g (0.l7M) of the alcohol product of Step 4, above, is added to 250ml of toluene. 20g of manganese dioxide are then added, and the mixture is heated slowly to reflux. When TLC indicates that no starting material remains (about l hour), the mixture is filtered through celite, washing with toluene then methylene chloride. The filtrate (organic phase containing the product) is evaporated to dryness to recover the title aldehyde product of this step.

Step 6: l-(2-ethoxyethenyl)-[3-(4-fluorophenyl)-l-isopropyl-2-indolizine]methanol

First, a reagent AVII is prepared by adding 0.8l5g (5.4mM) of cis-l-bromo-2-ethoxyethylene to 5ml of dry THF; the mixture is cooled to -70°, and 6.47ml of l.7M t-butyl-lithium (0.0llM) added dropwise. The resulting mixture is stirred for l hour at -70°.

To the above described reagent solution is added l.4g (4.9mM) of the aldehyde product of Step 5, above,in a minimum volume of dry THF. The resulting mixture is stirred, at -70°, for 2 hours (until none, or merely a trace of, starting material remains).

The mixture is added to ice-water, and extracted with ethyl acetate, the extracts combined, dried and evaporated to obtain as a residue crude title olefinic product of this step; which is used directly in Step 7, below.

Step 7: 3-[3-(4-fluorophenyl)-l-isopropyl-2-indolizinyl]-2-propenal (trans)

The olefinic crude product of Step 6, above, is added to a mixture of l60ml of THF, 40ml of water and lg of p-toluene sulfonic acid added. The resultant mixture is stirred for about l6 hours at RT. The mixture is added to water and extracted with ethyl acetate. The combined extracts are dried, then evaporated to dryness, then held for 2 hours under high vacuum. The product is then flash chromatographed.

Step 8: Ethyl 7-[3-(4-fluorophenyl)-l-isopropyl-2-indolizinyl]-5-hydroxy-3-oxo-hept-6-enoate (trans)

Reagent (Q) is first prepared by adding under a dry nitrogen atmosphere l.l3g (ll.2mM) of diisopropylamine to 50ml of dry THF; the mixture cooled to 0 to 5°, and 7.22ml (ll.2mM) of l.55M n-butyl-lithium (in hexane) is added dropwise thereto. The mixture is maintained at 0 to 5° for l5 minutes, then 0.728g (5.6mM) of ethyl acetoacetate (distilled) is added dropwise, and the mixture stirred at 0 to 5° for l hour.

The thus prepared reagent mixture is then cooled to -60° and 0.872lg (2.8mM) of the olefinic aldehyde product of Step 7, above, in a minimum volume of dry THF is added thereto and the mixture stirred at about -60° to -75° for l hour.

The reaction mixture is allowed to warm, ice-water added, and the mixture extracted with ethyl acetate. The combined extracts are dried, then evaporated to dryness to yield the crude title product of this step. This product is then flash distilled using ethyl acetate/methylene chloride (5:95 v/v) to obtain refined product (as a thick dark oil) for use in Step 9, below.

Step 9: Ethyl 7-[3-(4-fluorophenyl)-l-isopropyl-2-indolizinyl]-3,5-dihydroxy-hept-6-enoate (trans)

437mg (lmM) of the keto-ester product of Step 8, above, is mixed with 60ml dry THF and then l5ml of dry methanol. At RT, l.3ml of lM triethylborane in THF are added. Then is added 8ml of air. The mixture is stirred (at RT) for 2 hours.

The mixture is then cooled to -72°, 56mg of sodium borohydride added and the mixture stirred for 3 hours at -72°. 30mg additional sodium borohydride is added and the mixture stirred for l hour more at -72°. lml of acetic acid is then added dropwise. The temperature of the mixture is allowed to rise to RT. The mixture is then held at RT for l6 hours. 20ml of aqueous sodium bicarbonate solution is then added. Additional water is added and the product of this example is extracted with ethyl acetate. The combined extracts are dried and then evaporated to yield crude title product of this example. The product may be refined by chromatographing on prep plates, eluting with methanol/methylene chloride (5:95), as an oil; the erythro isomer predominating about 95:5.

EXAMPLE 9

7-[3-(4-fluorophenyl)-l-isopropyl-2-indolizinyl]-3,5-dihydroxy-6-heptenoic acid, sodium salt (trans)A compound Ia in which $R_1$ = 4-fluorophenyl, $R_2$ = isopropyl, A = (E)-CH=CH-and Z = a)

To l4ml of absolute ethanol is added l47.9mg of 7-[3-(4-fluorophenyl)-l-isopropyl-2-indolizinyl]-3,5-dihydroxy-6-heptenoic acid, ethyl ester (Example 9, above), and the solution cooled to 5°. There is then added thereto, dropwise 0.34ml of lN aqueous sodium hydroxide (o.34mM). The mixture is stirred for 2 hours while the temperature is allowed to rise. The mixture is evaporated to dryness, to obtain a residue, which is then held under high vacuum for 2 hours at 40°. Methylene chloride is added, the mixture warmed, filtered, cold ether is added dropwise, causing solids to precipitate. The solids are collected on by filtration, washed with ether, then dried at 40° under high vacuum to yield the title product, which decomposes at above 230°, the erythro isomer predominating about 9:l.

Adapting the procedure of Examples 8 and 9, the following compounds, Ia, are obtained in which A = (E)-CH=CH, Z = a), (the erythro isomer predominating about 9:l); (in the table, Et = ethyl, ip = isopropyl and ph = phenyl).

| Example No. | $R_1$ | $R_2$ | $R_8$ | Form | |
|---|---|---|---|---|---|
| 10 | 4-F-ph | H | Et | Oil | |
| 11 | 4-F-ph | H | Na | Solid | Decomp. > 200°C |
| 12 | ip | 4-F-ph | Et | Oil | |
| 13 | ip | 4-F-ph | Na | Solid | Decomp. > 220°C |

The following data were obtained for the preceding compounds. Unless otherwise stated the data are NMR spectra measured at 200mHz. Shifts are in ppm. relative to tetramethylsilane.

Abbreviations: s = singlet
d = doublet
dd = doublet of a doublet
t = triplet
q = quartet
Q = quintet
m = multiplet
br = broad
bs = broad singlet
dq = doublet of a quartet
dt = doublet of a triplet
Example No.

2 $CDCl_3$ : I.28(t,3H); 2.50(d,2H); 3.70(d,2H); 3.96 (s,3H); 4.I7(q,2H); 4.28(m,IH); 4.53(m,IH); 6.05 (q,IH): 6.74(q,IH); 7.I0(m,3H); 7.40(m,2H); 7.80 (m,IH); 8.32(m,IH),

3 $CDCl_3$ : I.45(s,9H); I.50(m,2H); I.65(m,6H); 2.39 (d,2H); 3.92(s,IH); 3.98(s,IH); 4.20(m,IH); 4.52 (m,IH); 4.86(m,IH); 5.9I(q,IH); 6.75(q,IH); 7.I0 (m,3H); 7.57(m,2H); 7.80(m,IH); 8.45(m,IH).

5A $CD_3OD$ : 2.33(m,2H); 3.30(m,2H); 3.93(s,3H); 4.02 (m,IH); 4.42(m,IH); 6.I0(q,IH); 6.76(q,IH); 7.I7 (m,3H); 7.46(m,2H), 7.87(m,IH); 8.23(m,IH).

5B (Ex.I) $CD_3OD$ : I.7I(d,6H); 2.30(m,2H); 3.94(m,IH); 4.39 (m,IH); 5.I3(m,IH); 5.80(q,IH); 6.75(q,IH); 7.II (m,3H); 7.43(m,2H); 7.78(m,IH); 8.20(m,IH).

5B (Ex.3) $CD_3OD$ : I.57(m,2H); I.68(d,6H); 2.30(m,2H); 3.97 (m,IH); 4.42(m,IH); 5.00(m,IH); 5.85(q,IH); 6.75 (q,IH); 7.I8(m,3H); 7.5I(m,2H); 8.09(m,IH); 8.25 (m,IH).

8 $CDCl_3$ : I.23(t,3H); I.42(d,6H); I.60(m,2H); 2.45 (m,2H); 2.93(bs,IH); 3.40(m,IH); 3.65(bs,IH); 4.I5(q,2H): 4.20(m,IH): 4.40(m,IH): 5.50(q,IH): 6.30(m,IH); 6.57(m,3H); 7.29(m,4H); 7.69(d,IH).

9 $CD_3OD$ : I.43(d,6H); I.65(m,2H); 2.30(m,2H); 3.42 (m,IH); 3.93(m,IH); 4.27(q,IH); 5.45(q,IH); 6.32 (m,IH); 6.57(m,2H); 7.30(m,5H); 7.72(d,IH).

I0 $CDCl_3$ : I.27(t,3H); I.72(m,2H); 2.48(d,2H); 3.03 (m,IH); 3.70(m,IH); 4.I5(q,2H); 4.26(m,IH); 4.50 (m,IH); 6.I5(q,IH); 6.50(m,4H); 6.62(s,IH); 7.29 (m,5H); 7.80(d,IH).

II $CD_3OD$ : I.70(m,2H); 2.30(m,2H); 4.02(m,IH); 4.33 (q,IH); 6.I2(q,IH); 6.50(m,4H); 6.63(s,IH); 7.35 (m,5H); 7.85(d.IH).

I2 $CDCl_3$ : I.25(t,3H); I.45(d,6H); I.60(m,2H); 2.45 (m,2H); 3.60(m,IH); 4.I7(q,2H); 4.20(m,IH); 4.45 (m,IH); 5.50(q,IH); 6.57(m,3H); 7.20(m,5H); 7.90 (d,IH).

I3 $CD_3OD$ : I.45(d,6H); I.65(m,2H); 2.29(m,2H); 3.69 (m,IH); 3.98(m,IH); 4.30(m,IH); 5.45(q,IH); 6.55 (m,3H); 7.20(m,5H); 8.05(d,IH).

## Claims

1. Compounds of Formula I

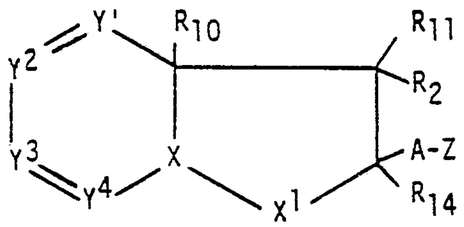

I

wherein

<u>either</u> :

one of $Y^1$ to $Y^4$ is -N-and the others are -CH-

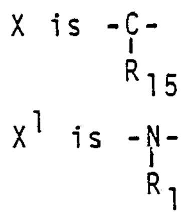

and $R_{10}$ and $R_{15}$, and $R_{11}$ and $R_{14}$ form bonds

$R_1$ is a primary or secondary $C_{1-6}$ alkyl;

$R_2$ is

a)

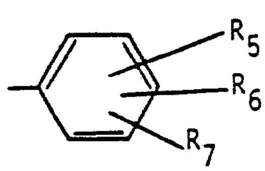

b) a primary or secondary $C_{1-6}$ alkyl,

c) $C_{3-6}$ cycloalkyl

d) phenyl-$(CH_2)_m$- wherein

$R_5$ is hydrogen, $C_{1-3}$ alkyl, n-butyl, i-butyl, t-butyl, $C_{1-3}$ alkoxy, n-butoxy, i-butoxy, trifluoromethyl, fluoro, chloro, phenoxy, or benzyloxy;

$R_6$ is hydrogen, $C_{1-3}$ alkyl, $C_{1-3}$ alkoxy, fluoro, chloro, trifluoromethyl, phenoxy or benzyloxy;

$R_7$ is hydrogen, $C_{1-2}$ alkyl, $C_{1-2}$ alkoxy, fluoro, or chloro;

m is 1,2 or 3;

with the proviso that not more than one of $R_5$ and $R_6$ is trifluoromethyl, not more than one of $R_5$ and $R_6$ is phenoxy, and not more than one of $R_5$ and $R_6$ is benzyloxy;

<u>or</u>:

$Y^1$ to $Y^4$ are -CH-

X is -N-

$X^1$ is 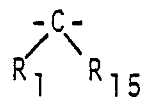

and $R_{10}$ and $R_{11}$, and $R_{14}$ and $R_{15}$ form bonds;

each of $R_1$ and $R_2$ is, independently, as defined under $R_2$ above, and $R_2$ may additionally be hydrogen;
A is

a)

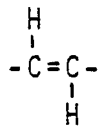 or 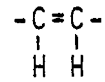

b) $-(CH_2)_n-$

wherein n is l,2 or 3

Z is

a) $\underset{OH}{CH}-CH_2-$ $\underset{OH}{CH}-CH_2-COOR_8$

b)

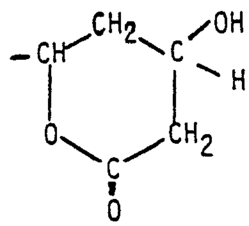

in which $R_8$ is hydrogen, $R_9$ or M;
wherein, $R_9$ is a physiologically acceptable and hydrolysable ester group and M is a pharmaceutically acceptable cation.

2. A compound according to Claim I wherein:

one of $Y^1$ to $Y^4$ is -N-, the remainder being -CH-

X is 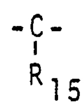

$X^1$ is 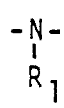

and $R_{10}$ and $R_{15}$, and $R_{11}$ and $R_{14}$ form bonds;

$R_1$ is a primary or secondary $C_{1-6}$ alkyl;

$R_2$ is

a)

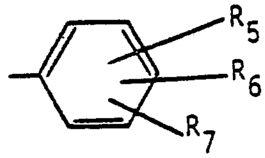

b) a primary or secondary $C_{1-6}$ alkyl
c) $C_{3-6}$ cycloalkyl
d) phenyl-$(CH_2)_m$- wherein

$R_5$ is hydrogen, $C_{1-3}$ alkyl, n-butyl, i-butyl, t-butyl, $C_{1-3}$ alkoxy, n-butoxy, i-butoxy, trifluoromethyl, fluoro, chloro, phenoxy or benzyloxy;
$R_6$ is hydrogen, $C_{1-3}$ alkyl, $C_{1-3}$ alko xy, fluoro, chloro, trifluoromethyl, phenoxy or benzyloxy;
$R_7$ is hydrogen, $C_{1-2}$ alkyl, $C_{1-2}$ alko xy, fluoro or chloro;
m is l,2 or 3;

with the proviso that not more than one of $R_5$ and $R_6$ is trifluoromethyl, not more than one of $R_5$ and $R_6$ is phenoxy, and not more than one of $R_5$ and $R_6$ is benzyloxy;

A is
a)

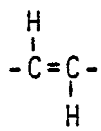

or - C = C -
H H

b) $-(CH_2)_n-$
wherein n is l,2, or 3;
Z is
a) C H-$CH_2$ - C H -$CH_2$ -$COOR_8$
O H O H

b)

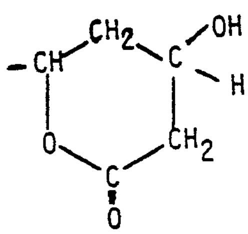

in which $R_8$ is hydrogen, $R_9$ or M;
wherein, $R_9$ is a physiologically acceptable and hydrolysable ester group and
M is a pharmaceutically acceptable cation.

3. A compound according to Claim l wherein:

each of $Y^1$ to $Y^4$ is -CH-
X is -N-

$X^1$ is 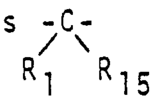

and $R_{10}$ and $R_{11}$, and $R_{14}$ and $R_{15}$ form bonds;

each of $R_1$ and $R_2$ is, independently

a)

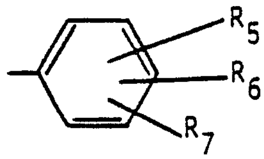

b) a primary or secondary $C_{1-6}$ alkyl

c) $C_{3-6}$ cycloalkyl

d) phenyl-$(CH_2)_m$-

and $R_2$ may additionally be hydrogen

wherein

$R_5$ is hydrogen, $C_{1-3}$ alkyl, n-butyl, i-butyl, t-butyl, $C_{1-3}$ alkoxy, n-butoxy, i-butoxy, trifluoromethyl, fluoro, chloro, phenoxy, or benzyloxy;

$R_6$ is hydrogen, $C_{1-3}$ alkyl, $C_{1-3}$ alko xy, fluoro, or chloro;

$R_7$ is hydrogen, $C_{1-2}$ alkyl, $C_{1-2}$ alko xy, fluoro, or chloro;

m is I,2 or 3;

A is

a)

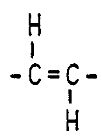

or 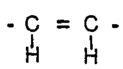

b) $-(CH_2)_n-$

wherein n is I,2 or 3;

Z is

a) 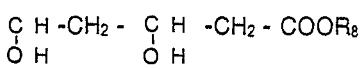

b)

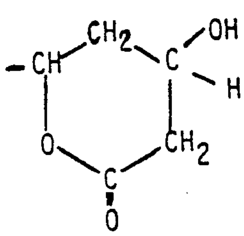

in which $R_8$ is hydrogen, $R_9$ or M

wherein, $R_9$ is a physiologically acceptable and hydrolysable ester group and M is a pharmaceutically acceptable cation.

4. A compound according to Claim I in which the substituents have the following meanings:

either $Y^1$ or $Y^4$ is -N-and the others are -CH-

X is 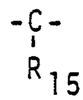

$X^1$ is 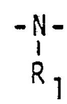

and $R_{10}$ and $R_{15}$, and $R_{11}$ and $R_{14}$ form bonds;
A is

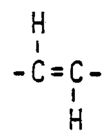

Z is 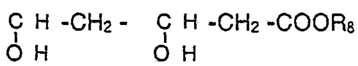

$R_1$ is a $C_{1-2}$ alkyl or isopropyl

$R_2$ is 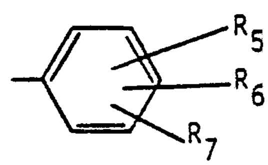

wherein one of $R_5$, $R_6$ and $R_7$ is fluoro and the others are hydrogen.

5. A compound according to Claim I in which the substituents have the following meanings:

each Y is -CH-
X is -N-

$X^1$ is 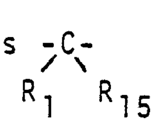

and $R_{10}$ and $R_{11}$, and $R_{14}$ and $R_{15}$ form bonds;

A is

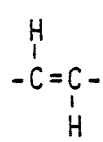

Z is 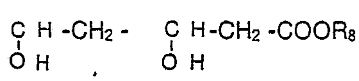

each of $R_1$ and $R_2$ is, independently, isopropyl or

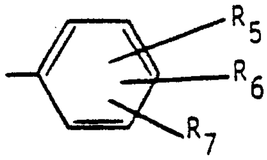

and $R_2$ may additionally be hydrogen,

wherein one of $R_5$, $R_6$ and $R_7$ is fluoro and the others are hydrogen.

6. A compound according to Claim I in which:

i) $Y^4$ is -N-and the remainder are -CH-

X is 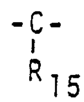

$X^1$ is 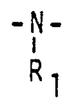

and $R_{10}$ and $R_{15}$, and $R_{11}$ and $R_{14}$ form bonds;

$R_1$ is isopropyl
$R_2$ is 4-fluorophenyl
A is

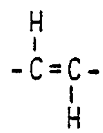

Z is a) as defined above.

ii) $Y^1$ to $Y^4$ are -CH-
X is -N-

$X^1$ is 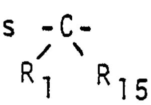

and $R_{10}$ and $R_{11}$, and $R_{14}$ and $R_{15}$ form bonds;

$R_1$ is 4-fluorophenyl
$R_2$ is isopropyl
A is

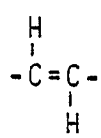

Z is a) as defined above.

7. A compound according to Claim 6 in which $R_8$ is M as defined above.

8. A compound according to Claim 7 in which M is sodium.

9. A pharmaceutical composition comprising a compound according to Claim I as appropriate in free acid form or in the form of a physiologically hydrolysable and acceptable ester or lactone thereof or in pharmaceutically acceptable salt form, together with a pharmaceutically acceptable diluent or carrier.

I0. A compound according to Claim I as appropriate in free acid form or in the form of a physiologically hydrolysable and acceptable ester or a lactone thereof or in pharmaceutically acceptable salt form for use as a pharmaceutical.

II. A compound according to Claim I as appropriate in free acid form or in the form of a physiologically hydrolysable and acceptable ester or a lactone thereof or in pharmaceutically acceptable salt form for use in inhibiting cholesterol biosynthesis or treating atherosclerosis.

I2. A process for preparing a compound according to Claim I which comprises hydrolysing a compound of Formula I in ester or lactone form or esterifying or lactonising a compound of Formula I in free acid form and when a free carboxyl group is present recovering the compound obtained in free acid form or in the form of a salt.

I3. A process for preparing a compound according to Claim I which comprises:

a) Reducing a compound of formula AII:

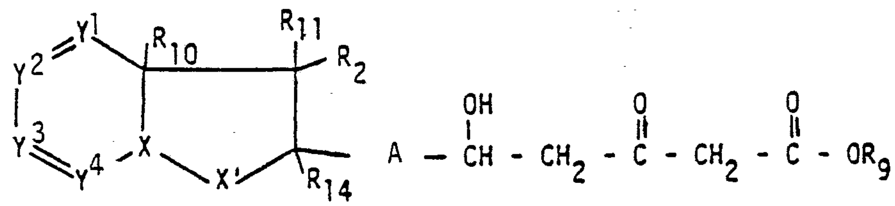

AII

b) Deprotecting a compound of formula BII:

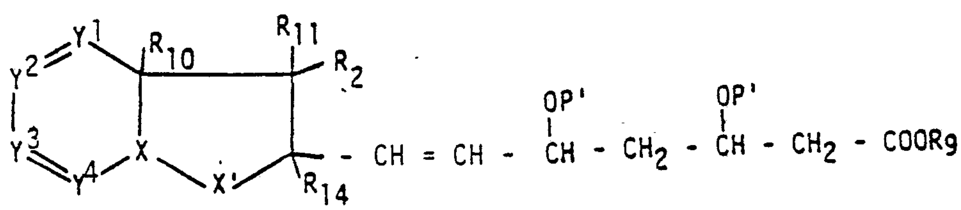

BII

c) hydrolysing a compound of Formula I in the form of an ester or a lactone or

d) esterifying or lactonising a compound of Formula I in free acid form and when a free carboxyl group is present, recovering the compound obtained in free acid form or in the form of a salt.